# EUROPÄISCHE PATENTSCHRIFT

(11) **EP 0 531 256 B1**
(45) Veröffentlichungstag und Bekanntmachung des Hinweises auf die Patenterteilung: **23.04.1997**
(21) Anmeldenummer: 92810654.1
(22) Anmeldetag: 26.08.1992
(51) Int. Cl.: C07H 15/02, C07H 15/04, C07H 15/20

(54) **Verfahren zur Herstellung von Glykosiden**
Method for preparing glycosides
Méthode pour la préparation des glycosides

(30) Priorität: 04.09.1991 CH 2603/91
(43) Veröffentlichungstag der Anmeldung: 10.03.1993
(73) Patentinhaber: Novartis AG, 4058 Basel (CH)
(72) Erfinder: Ernst, Beat, Dr., CH-4312 Magden (CH); Heneghan, Michael, Dr., Salford M6 7QN (GB); Hafner, Andreas, Dr., CH-4402 Frenkendorf (CH)

(56) Entgegenhaltungen:
- EP-A- 0 168 723
- US-A- 3 972 868
- ULLMANNS ENCYKLOP DIE DER TECHNISCHEN CHEMIE, 4. Auflage, Band 16, 1978, Verlag Chemie GmbH, Seiten 587-607

## Beschreibung

Die Erfindung betrifft ein Verfahren zur Herstellung von Glykosiden durch die Umsetzung von geschützten Zuckern mit einer anomeren Hydroxylgruppe und einem Aglykon aus der Gruppe Alkohol und einem geschützten Zucker mit einer nicht-anomeren Hydroxylgruppe in einem inerten Lösungsmittel bei Temperaturen von bevorzugt -20 bis 250 °C in Gegenwart katalytischer Mengen von Metallkomplexsalzen.

Bei der Herstellung von Glykosiden sind Verfahren bekannt, bei denen zunächst die anomere Hydroxylgruppe durch Substitution mit -F[K.C. Nicolaou et al., J. Amer. Chem. Soc. 107, 5556 (1985)], -Br [W. Koenig et al., Chem. Ber. 34, 957 (1901)] oder -O-C(=NH)CCl₃ [R.R. Schmidt et al., Angew. Chemie, Int. Ed. Engl. 25, 725 (1986)] aktiviert und dann in Gegenwart von Lewissäuren oder stöchiometrischen Mengen an Silber- oder Quecksilbersalzen mit einem Aglykon, zum Beispiel einem Alkohol, umgesetzt wird. Auf Grund der aufwendigen Synthese und der physiologisch bedenklichen Metallsalze sind die Verfahren für eine industrielle Produktion nicht geeignet.

Die direkte Glykosilierung der anomeren Hydroxylgruppe von ungeschützten Zuckern (D-Glucose) mit Methanol als Aglykon wird von E. Fischer et al. in Ber. 26, Seite 2400, (1893) und Ber. 28, Seite 1145, (1895) beschrieben. Die thermodynamisch kontrollierte Reaktion verläuft relativ langsam und liefert stets Gemische von Methyl-α- und Methyl-β -D-Glucopyranosid und Methyl-α- und -β-D-Glucofuranosid.

Es wurde nun überraschend gefunden, dass die direkte Glykosilierung auch durch die katalytische Wirkung von Metallkomplexsalzen in hohen Ausbeuten und relativ kurzen Reaktionszeiten zu den gewünschten Glykosiden führt. Zudem werden oft hohe Anteile der α- oder β-Form erzielt. Auf Grund der katalytischen Mengen an Metallkomplexen stehen auch keine ökologischen Bedenken einer Durchführung im industriellen Masstab entgegen.

Gegenstand der Erfindung ist ein Verfahren zur Herstellung von Glykosiden durch die Umsetzung von Zuckern mit einer anomeren Hydroxylgruppe und einem Aglykon, das dadurch gekennzeichnet ist, dass man in einem inerten Lösungsmittel einen geschützten Zucker, der eine anomere Hydroxylgruppe enthält, mit einem Aglykon aus der Gruppe a) aliphatische Alkohole, cycloaliphatische Alkohole, aromatische oder aromatisch-aliphatische Alkohole, und b) geschützte Zucker mit einer nicht-anomeren Hydroxylgruppe entweder je alleine oder je zusammen mit einem Orthoester, in Gegenwart katalytischer Mengen eines Metallkomplexsalzes aus (1) einem Metallkation eines Metalls aus der zweiten bis fünften Hauptgruppe, der ersten bis achten Nebengruppe oder der Lanthaniden des Periodensystems der Elemente, (2) einem oder mehreren, gleichen oder verschiedenen, ein- oder mehrzähnigen Liganden entsprechend der Koordinationszahl des Metallkations, (3) gegebenenfalls einem nukleophilen Anion aus der Gruppe Halogenid, Pseudohalogenid, C₁-C₈-Alkoholat oder unsubstituiertes oder mit C₁-C₄-Alkyl substituiertes Phenolat, sekundäres Amido mit 2 bis 12 C-Atomen, Bis-[(Tri-C₁-C₆-Alkyl)silyl]amido oder unsubstituiertes oder mit C₁-C₄-Alkyl substituiertes Cyclopentadienyl, und (4) einem nicht-nulkeophilen Anion aus der Gruppe Anion einer Sauerstoffsäure, BF₄, PF₆, AsF₆ oder SbF₆ entsprechend der Wertigkeit des komplexierten Metallkations, umsetzt.

Die im erfindungsgemässen Verfahren verwendeten geschützten Zucker sind bekannt, nach bekannten Verfahren herstellbar und viele der Zucker sind kommerziell erhältlich. Es kann sich zum Beispiel um geschützte Mono- und Oligosaccharide handeln, wie zum Beispiel Mono-, Di-, Tri-, Tetra- und Pentasaccharide.

In einer bevorzugten Ausführungsform handelt es sich bei den geschützten Mono- und Oligosacchariden um Aldosen oder Ketosen mit einer anomeren Hydroxylgruppe.

In einer besonders bevorzugten Ausführungsform handelt es sich bei dem geschützten Monosaccharid um Aldopyranosen, Aldofuranosen, Ketopyranosen oder Ketofuranosen mit einer anomeren Hydroxylgruppe.

Insbesondere ist die Aldopyranose D-Ribose, D-Arabinose, D-Xylose, D-Lyxose, D-Allose, D-Altrose, D-Glucose, D-Mannose, D-Gulose, D-Iodose, D-Galaktose oder D-Talose; die Aldofuranose D-Erythrose, D-Threose, D-Ribose, D-Arabinose, D-Xylose, D-Lyxose, D-Allose, D-Altrose, D-Glucose, D-Mannose, D-Gulose, D-Iodose, D-Galaktose oder D-Talose ; die Ketopyranose D-Piscose, D-Fructose, D-Sorbose oder D-Tagatose; und die Ketofuranose D-Piscose, D-Fructose, D-Sorbose oder D-Tagatose, deren Hydroxylgruppen ausser der anomeren Hydroxylgruppe geschützt sind.

Insbesondere ist das Disaccharid Trehalose, Sophorose, Kojibiose, Laminaribiose, Maltose, Cellobiose, Isomaltose, Gentibiose, Saccharose, Raffinose und Lactose, deren Hydroxylgruppen ausser der anomeren Hydroxylgruppe geschützt sind.

Geschützte Mono- und Oligosaccharid-Derivate mit einer anomeren Hydroxylgruppe sind bekannt, nach bekannten Verfahren herstellbar und teilweise käuflich. Es kann sich zum Beispiel um geschützte Desoxyzucker, Aminozucker, Thiozucker, Zuckersäuren oder Ester von Zuckersäuren, die eine anomere Hydroxylgruppe enthalten, wie zum Beispiel 2-Deoxyzucker, 2-Thiozucker, 2-Aminozucker, Gluconsäuren und deren Ester, bevorzugt deren C₁-C₄-Alkylester handeln.

Geschützt bedeutet, dass die Hydroxylgruppen der Mono und Oligosaccharide beziehungsweise der Mono- und Oligosaccharidderivate ausser der anomeren Hydroxylgruppe mit einer abspaltbaren Schutzgruppe derivatisiert sind. Solche Schutzgruppen und Verfahren zur Derivatisierung sind in der Zuckerchemie allgemein bekannt. Beispiele für solche Schutzgruppen sind: lineares oder verzweigtes C₁-C₈-, besonders C₁-C₄-Alkyl, zum Beispiel Methyl, Ethyl, n- und i-Propyl, n-, i- und t-Butyl; C₇-C₁₂-Aralkyl, zum Beispiel Benzyl; Trialkylsilyl mit 3 bis 20, besonders 3 bis 12 C-Atomen, zum Beispiel Trimethylsilyl, Triethylsilyl, Tri-n-propylsilyl, i-Propyl-dimethylsilyl, t-Butyl-dimethylsilyl, t-Butyl-diphenylsilyl, n-Octyl-dimethylsilyl, (1,1,2,2-Tetramethylethyl)-dimethylsilyl; substituierte Methylidengruppen, die durch Acetal- beziehungsweise Ketalbildung von benachbarten OH-Gruppen der Zukker beziehungsweise Zuckerderivate mit Aldehyden und Ketonen erhältlich sind, die vorzugsweise 2 bis 12 beziehungsweise 3 bis 12 C-Atome enthalten, zum Beispiel C₁-C₁₂, bevorzugt C₁-C₆- und besonders C₁-C₄-Alkyliden (Ethyliden, 1,1- oder 2,2-Propyliden, 1,1- oder 2,2-Butyliden) oder Benzyliden; C₂-C₁₂-, besonders C₂-C₈-Acyl, wie zum Beispiel Acetyl, Propanoyl, Butanoyl, Pentanoyl, Hexanoyl und Benzoyl; R-SO₂-, worin R C₁-C₁₂-Alkyl, besonders C₁-C₆-Alkyl, C₅- oder C₆-Cycloalkyl, Phenyl, Benzyl, C₁-C₁₂- und besonders C₁-C₄-Alkylphenyl, oder C₁-C₁₂- und besonders C₁-C₄-Alkylbenzyl bedeutet, zum Beispiel Methyl-, Ethyl-, Propyl-, Butyl-, Phenyl-, Benzyl- und p-Methylphenylsulfonyl.

Die Orthoester leiten sich bevorzugt von einer C₁-C₄-Carbonsäure ab, zum Beispiel Ameisensäure, Essigsäure, Propionsäure oder Buttersäure. Besonders bevorzugt sind Ameisensäureorthoester. Bevorzugte Orthoester sind solche aus diesen Säuren und einem linearen oder verzweigten C₁-C₂₀-Alkanol, C₂-C₂₀-Alkenol mit einer nicht-vinylischen Alkoholgruppe, einem mono- oder polycycloaliphatischen oder einem mono- oder polycycloheteroaliphatischen (mit den Heteroatomen O, S und N) C₃-C₈-Alkohol, einem aromatischen C₆-C₂₀-Alkohol, oder einem aromatisch-aliphatischen C₇-C₂₀-Alkohol, die unsubstituiert oder mit C₁-C₆-Alkyl, C₁-C₆-Alkoxy oder -Alkylthio, Phenyloxy, C₁-C₄-Alkyl- oder -Alkoxyphenyloxy, Benzyl, Benzyloxy oder C₁-C₄-Alkyl- oder -Alkoxybenzyloxy substituiert sind. Orthoester mit Alkoholen sind bevorzugt.

Beispiele für Alkanole, die bevorzugt 1 bis 12 C-Atome enthalten, sind Methanol, Ethanol, n- und i-Propanol, n-, i- oder t-Butanol, Pentanol, Hexanol, Heptanol, Octanol, Nonanol, Decanol, Undecanol, Dodecanol, Tetradecanol, Hexadecanol, Octadecanol, Eicosanol.

Beispiele für Alkenalkanole sind Allylalkohol, But-1-en-4-ol, But-2-en-4-ol, Pent-1- oder -2-en-5-ol und Hex-1-en-6-ol.

Beispiele für cycloaliphatische Alkohole, die bevorzugt 1 bis 4 Ringe enthalten, sind Cyclopropanol, Cyclobutanol, Cyclopentanol, Cyclohexanol, Cycloheptanol und Cyclooctanol. Bei den cycloaliphatischen und cycloheteroaliphatischen Alkoholen kann es sich auch um kondensierte Ringsysteme handeln, wie sie vielfach in Naturprodukten vorkommen.

Als aromatische Alkohole kommen besonders Phenole und Naphthole in Frage.

Als aromatische-aliphatische Alkohole kommen bevorzugt Phenylalkanole mit bevorzugt 1 bis 18, besonders bevorzugt 1 bis 12 C-Atomen in der Alkanolgruppe in Frage, zum Beispiel Benzylalkohol, 1-Phenylethan-2-ol, 1-Phenylpropan-3-ol, 1-Phenyloctan-8-ol.

Die beispielhaft aufgezählten und bevorzugten Alkohole können unsubstutuiert oder wie zuvor definiert substituiert sein. Eine bevorzugte Gruppe von Alkoholen für die Orthoester, besonders bevorzugt für die Orthoameisensäureester, sind C₁-C₄-Alkanole, Allylalkohol , Benzylalkohol und Phenol.

Als Aglykon a) geeignete Alkohole sind ebenfalls die für die Orthoester genannten Alkohole. Die aliphatischen Alkohole sind bevorzugt. Eine bevorzugte Gruppe der Aglykone a) sind Alkanole und Alkenole mit 1 bzw. 2 bis 18, besonders bevorzugt 1 bzw. 2 bis 12 C-Atomen, mono- bis tetracyclische Cycloaliphaten mit 3 bis 20, besonders bevorzugt 5 bis 20 C-Atomen, Phenol und Benzylalkol, die unsubstituiert oder mit C₁-C₆-Alkyl, C₁-C₆-Alkoxy oder -Alkylthio, Phenyloxy, C₁-C₄-Alkyl- oder -Alkoxyphenyloxy, Benzyl, Benzyloxy oder C₁-C₄-Alkyl- oder -Alkoxybenzyloxy substituiert sind.

Als Aglykone b) sind die zuvor erwähnten geschützten Zucker bzw. Zuckerderivate geeignet, deren anomere Hydroxylgruppe geschützt ist und die eine freie Hydroxylgruppe enthalten. Bevorzugt enthalten diese Zucker eine Hydroxymethylgruppe. Für diese geschützten Zucker bzw. Zuckerderivate gelten die zuvor beschriebenen Bevorzugungen.

Katalytische Mengen bedeutet bevorzugt eine Menge von 0,01 bis 20 Mol-%, besonders bevorzugt 0,01 bis 15 Mol-% und ganz besonders bevorzugt 0,01 bis 10 Mol-%, bezogen auf die Menge des geschützten Zuckers bzw. Zuckerderivates.

Erfindungsgemäss zu verwendende Metallkomplexe der Formel I sind in grosser Vielzahl bekannt oder nach bekannten und analogen Verfahren herstellbar.

Die Metallkomplexsalze können ein- oder mehrkernig, zum Beispiel ein- oder zweikernig sein. Bevorzugt sind die Metallkomplexsalze einkernig. Die Metallkationen leiten sich bevorzugt von folgenden Metallen aus den genannten Gruppen des Periodensystems der Elemente ab: Mg, Ca, Sr, Ba; B, Al, Ga, In; Sn, Pb; Sb, Bi; Cu, Ag, Au; Zn, Cd, Hg; Sc, Y, La; Ti, Zr, Hf; V, Nb, Ta; Cr, Mo, W; Mn; Fe, Co, Ni, Ru, Rh, Pd, Os, Ir, Pt; und den Lanthanidenmetallen Ce, Pr, Nd, Pm, Sm, Eu, Gd, Tb, Dy, Ho, Er, Tm, Yb und Lu. Bevorzugter sind Metallkationen der Metalle Mg, Ca, Sr, B, Al, In, Sn, Pb, Bi, Cu, Ag, Au, Zn, Ti, Zr, Hf, V, Nb, Cr, Mo, W, Mn, Fe, Co, Ni, Ru, Rh, Pd, Ir, Pt, Ce, Nd, Sm, Eu und Yb. Noch bevorzugter sind Metallkationen der Metalle Mg, Ca, B, In, Sn, Pb, Cu, Ag, Au, Zn, Ti, Zr, V, Cr, Mo, W, Mn, Fe, Co, Ni, Ru, Rh, Pd, Ir, Pt, Ce, Nd und Yb. Besonders bevorzugt sind Metallkationen der Metalle Mg, B, Sn, Cu, Ag, Zn, Ti, Zr, Mn, Ni, Ru, Rh, Pd, Ir, Pt und Ce. Ganz besonders bevorzugt sind Metallkationen der Metalle Mg, B, Cu, Ag, Zn, Rh, Pd, Pt, und Ce.

Der Ligand kann achiral oder chiral sein. Bevorzugt sind ein- bis dreizähnige Liganden. Solche Liganden sind ebenfalls in grosser Vielzahl in der Literatur beschrieben. Die mehrzähnigen Liganden mit den komplexbildenden Gruppen bilden mit dem Metallkation bevorzugt einen fünf- bis siebengliedrigen Ring, besonders bevorzugt einen fünf- oder sechsgliedrigen Ring. Die komplexbildenden Gruppen der mehrzähnigen Liganden sind daher bevorzugt in den 1,2-, 1,3- oder 1,4-Stellungen einer Kohlenstoffkette mit 2 bis 4, besonders bevorzugt 2 oder Kohlenstoffatomen gebunden, wobei es sich um lineare oder mono- oder bicyclische Verbindungen handeln kann. Diese Verbindungen können 2 bis 30, bevorzugt 2 bis 20, insbesondere bevorzugt 2 bis 12 C-Atome enthalten, ohne die C-Atome in den komplexbildenden Gruppen.

Bei den Liganden kann es sich zum Beispiel um schwach koordinierende organische-Liganden handeln, die 1 bis 20, bevorzugt 1 bis 12 C-Atomen enthalten und die zur Koordination befähigte Heteroatome oder Heterogruppen enthalten, zum Beispiel -OH, -CN, -CHO, -CO-, -O-, -C(O)OR₀ und P(O-)₃, worin R₀ der Rest eines C₁-C₁₂-Alkohols ist, zum Beispiel von C₁-C₁₂-Alkanolen, C₅-C₈-Cycloalkanolen, Phenol, (C₁-C₆-Alkyl)phenolen, Benzylalkohol oder (C₁-C₆-Alkyl)benzylalkoholen. Als anorganischer Ligand kommt zum Beispiel H₂O in Frage. Bei diesen Liganden kann es sich bevorzugt um H₂O oder organische Verbindungen handeln, die auch im erfindungsgemässen Verfahren als Lösungsmittel verwendet werden können, und die die Metallkationen der Metallkomplexe zu solvatisieren vermögen. Die organischen Liganden können ein oder mehrere, gleiche oder verschiedene Heteroatome und Heterogruppen enthalten, wobei auch stark koordinierende Verbindungen umfasst werden können, zum Beispiel β-Diketone (Acetylaceton), β-ketocarbonsäureester (Acetylessigsäureethylester), β-Dialdehyde (Malondialdehyd), und β-Ketoaldehyde(Acetylacetaldehyd).

Beispiele für solche Liganden sind neben Wasser: Alkohole mit 1 bis 12 C-Atomen (Methanol, thanol, Ethanol, n- oder i-Propanol, n-, i- oder t-Butanol, Pentanol, Hexanol, Octanol, Dodecanol, Benzylalkohol); Nitrile und Isonitrile (Acetonitril, Propionitril, Butyronitril, Benzonitril, Phenylessigsäurenitril und entsprechende Isonitrile); Aldehyde (Formaldehyd, Acetaldehyd, Propionaldehyd, Benzaldehyd, Pivaldehyd); Ketone (Aceton, Methylethylketon, Diethylketon, Methylisobutylketon), C₁-C₄-Alkylester von bevorzugt aliphatischen Carbonsäuren (Methyl-, Ethyl-, Propyl oder Butyl-, bevorzugt Methylester von Ameisen-, Essig-, Propion- oder Buttersäure, bevorzugt von Essigsäure); Ether (Dimethylether, Diethylether, Dibutylether, Ethylenglykoldiethyl- oder -dimethylether, Tetrahydrofuran, Dioxan); Phosphite [(Trifluormethyl)phosphit]. Eine bevorzugte Gruppe sind Acetonitril, Benzonitril, Methanol, Ethanol, Phenol, Aceton, Pivaldehyd, Essigsäureethylester, Diethylether, Tetrahydrofuran, Dioxan und Wasser.

Die Metallkomplexsalze können nur die oben erwähnten schwach koordinierenden Liganden, nur stark kordinierende Liganden oder beide Ligandenarten enthalten, wobei die Zusammensetzung von der Koordinationszahl des Metallkations, die vorzugsweise 2 bis 8, bevorzugter 2 bis 6, und insbesondere bevorzugt 2, 4 oder 6 beträgt, von der Zähnigkeit der Liganden und von der An- beziehungsweise Abwesenheit nukleophiler Anionen abhängt. Stark koordinierende Liganden sind bevorzugt organische Verbindungen, die 2 bis 30, bevorzugt 2 bis 20 und besonders bevorzugt 2 bis 16 C-Atome und als komplexbildende Gruppen ein oder mehrere, gleiche oder verschiedene, gegebenenfalls substituierte Amino-, Phosphino-, Phosphonit-, Arsino- oder Stibinogruppen enthalten. Geeignete Substituenten für diese Gruppen sind zum Beispiel C₁-C₁₂-, bevorzugt C₁-C₆-Alkyl, C₅- oder C₆-Cycloalkyl, Phenyl oder Benzyl, die unsubstituiert oder mit C₁-C₄-Alkyl oder C₁-C₄-Alkoxy substituiert sind. Einige bevorzugte Beispiele sind Methyl, Ethyl, Cyclopentyl, Cyclohexyl, Methylcyclohexyl, Phenyl, Methylphenyl, Methoxyphenyl, Benzyl, Methylbenzyl und Methoxybenzyl. Besonders bevorzugt sind Methyl, Cyclohexyl, Phenyl, Methylphenyl und Methoxyphenyl. Die stark koordinierenden Liganden können einzähnig bis vierzähnig sein.

Beispiele für einzähnige stark koordinierende Liganden sind insbesondere tertiäre Amine, Phosphine, Arsine und Stibine, zum Beispiel der Formel XR₁R₂R₃, worin X für N, P, As oder Sb steht und R₁, R₂ und R₃ unabhängig voneinander C₁-C₁₂-, bevorzugt C₁-C₆-Alkyl, C₅- oder C₆-Cycloalkyl, Phenyl oder Benzyl bedeuten, oder R₁ und R₂ zusammen Tetramethylen, Pentamethylen oder 3-Oxa-1,5-pentylen darstellen und R₃ die zuvor angegebene Bedeutung hat, die unsubstituiert oder mit C₁-C₄-Alkyl oder C₁-C₄-Alkoxy substituiert sind. Einige bevorzugte Beispiele sind Methyl, Ethyl, Cyclopentyl, Cyclohexyl, Methylcyclohexyl, Phenyl, Methylphenyl, Methoxyphenyl, Benzyl, Methylbenzyl und Methoxybenzyl. Besonders bevorzugt sind Methyl, Cyclohexyl, Phenyl, Methylphenyl und Methoxyphenyl. Bevorzugt haben R₁, R₂ und R₃ die gleiche Bedeutung. Einige Beispiele für bevorzugte Amine, Phosphine, Arsine und Stibine sind Trimethyl-, Triethyl-, Tri-n-propyl-, Tri-n-butyl-, Triphenyl-, Tri(methylphenyl)-, Methyldiphenyl- und Phenyldimethylamin, -phosphin, -arsin oder -stibin. Bei den tertiären Aminen kann es sich auch um Pyridin oder N-(C₁-C₆-alkylierte) cyclische Amine handeln, zum Beispiel N-Methylmorpholin, N-Methylpiperidin und N-Methylpyrrolidin. Bevorzugte einzähnige Liganden sind die Phosphine.

Die komplexbildenden Gruppen in den stark koordinierenden mehrzähnigen Liganden können den Formeln -NR₁R₂, -PR₁R₂, -OPR₁R₂, -AsR₁R₂ oder -SbR₁R₂ entsprechen, wobei die Gruppe -PR₁R₂ bevorzugt ist. R₁ und R₂ können unabhängig voneinander C₁-C₁₂, bevorzugt C₁-C₆-Alkyl, C₅- oder C₆-Cycloalkyl, Phenyl oder Benzyl sein, die unsubstituiert oder mit C₁-C₄-Alkyl oder C₁-C₄-Alkoxy substituiert sind. Einige bevorzugte Beispiele sind Methyl, Ethyl, Cyclopentyl, Cyclohexyl, Methylcyclohexyl, Phenyl, Methylphenyl, Methoxyphenyl, Benzyl, Methylbenzyl und Methoxybenzyl. Besonders bevorzugt sind Methyl und Phenyl. Bevorzugte komplexbildende Gruppen sind Amino- und besonders bevorzugt Phosphinogruppen. Die mehrzähnigen Liganden können gleiche oder verschiedene komplexbildende Gruppen der Formeln -NR₁R₂, -PR₁R₂, -OPR₁R₂, -AsR₁R₂ oder -SbR₁R₂ enthalten, zum Beispiel -NR₁R₂ und -PR₁R₂, oder -PR₁R₂ und -OPR₁R₂, oder -PR₁R₂ und -AsR₁R₂.

Die komplexbildenden Gruppen der mehrzähnigen, vorzugsweise zwei- bis vierzähnigen Liganden sind bevorzugt in den 1,2-, 1,3- oder 1,4- Stellungen einer Kohlenstoffkette mit 2 bis 4 C-Atomen gebunden. Die komplexbildenden Gruppen der drei- und vierzähnigen Liganden sind bevorzugt in den 1,2-, 1,3- oder 1,4- Stellungen einer aliphatischen C₂-C₄-Kohlenstoffkette gebunden.

Ein Beispiel für dreizähnige Liganden sind Phosphine der Formel R₄C(CH₂PR₁R₂)₃, worin R₁ und R₂ die zuvor angegebenen Bedeutungen hat, einschliesslich der Bevorzugungen, und R₄ für H, C₁-C₄-Alkyl, Phenyl oder Benzyl steht. Besonders bevorzugt sind CH₃-C[CH₂P(C₆H₅)₂]₃ und Bis-[(2-diphenylphosphino)ethyl]phenylphosphin. Weitere Beispiele für dreizähnige Liganden sind N,N',N"-pentaalkylierte Diethylentriamine, wobei die Alkylgruppen bevorzugt 1 bis 4 C-Atome enthalten. Ein Beispiel ist N,N',N"-Pentamethyl-diethylentriamin.

Beispiele für vierzähnige Liganden sind C[CH₂P(C₆H₅)]₄, Tri[(2-diphenylphosphino)ethyl]phosphin, Tri[(2-dimethylamino)ethyl]amin und N,N',N",N‴-Hexamethyl-triethylentetraamin

Zweizähnige Liganden sind in grosser Vielfalt bekannt und sind von H. Brunner in Topics in Stereochemistry, 18, Seiten 129 bis 247 (1988) beschrieben. Sie können gleiche oder verschiedene der zuvor erwähnten komplexbildenden Gruppen enthalten und es kann sich um aliphatische, cycloaliphatische, cycloheteroaliphatische, aromatische oder heteroaromatische Verbindungen mit bevorzugt 2 bis 30, besonders bevorzugt 2 bis 20 C-Atomen handeln (ohne die C-Atome in den komplexbildenden Gruppen), wobei die komplexbildenden Gruppen in den aliphatischen Verbindungen in 1,2-, 1,3- oder 1,4-Stellung und in den cyclischen Verbindungen in 1,2- oder 1,3-Stellung gebunden sind, und wobei die Heteroatome aus der Gruppe O, S und N ausgewählt sind und wobei bevorzugt 1 oder 2 enthalten sind, und wobei die komplexbildenden Gruppen direkt oder über eine -CR₅R₆-Gruppe an das Grundgerüst gebunden sind, und R₅ und R₆ unabhängig voneinander H, C₁-C₄-Alkyl, Phenyl oder Benzyl bedeuten. Als komplexbildende Gruppen kommen zum Beispiel solche der Formeln -NR₁R₂, -PR₁R₂, -OPR₁R₂, -AsR₁R₂ oder -SbR₁R₂ in Frage, wobei die Gruppe -PR₁R₂ bevorzugt ist. R₁ und R₂ können unabhängig voneinander C₁-C₁₂, bevorzugt C₁-C₆-Alkyl, C₅- oder C₆-Cycloalkyl, Phenyl oder Benzyl sein, die unsubstituiert oder mit C₁-C₄-Alkyl oder C₁-C₄-Alkoxy substituiert sind. Einige bevorzugte Beispiele sind Methyl, Ethyl, Cyclopentyl, Cyclohexyl, Methylcyclohexyl, Phenyl, Methylphenyl, Methoxyphenyl, Benzyl, Methylbenzyl und Methoxybenzyl. Besonders bevorzugt sind Methyl und Phenyl. Bevorzugte komplexbildende Gruppen sind Amino- und besonders bevorzugt sind Phosphinogruppen. Weitere komplexbildende Gruppen sind besonders sp²-Stickstoffatome in ungesättigten heterocyclischen Verbindungen, wie sie zum Beispiel in Pyridinen, Oxazolinen und Pyrazolen vorliegen, sowie gegebenenfalls über eine Kohlenstoffkette mit 1 bis 3 C-Atomen an die obigen Grundgerüste gebundene 2 N-(C₁-C₆-alkylierte), N-phenylierte oder N-benzylierte Imingruppen, oder eine an zum Beispiel Pyridine, Oxazoline oder Pyrazole gebundene solche Imingruppe.

In einer bevorzugten Ausführungsform können die zweizähnigen Liganden der Formel I entsprechen,

R₁R₂X-Y-X'R₁R₂ (I),

worin X und X' unabhängig voneinander für N, P, As, Sb oder -OP stehen, wobei P und -OP bevorzugt sind. X und X' sind bevorzugt gleich. Besonders bevorzugt stellt X P dar. R₁ und R₂ können unabhängig voneinander C₁-C₁₂, bevorzugt C₁-C₆-Alkyl, C₅- oder C₆- Cycloalkyl, Phenyl oder Benzyl sein, die unsubstituiert oder mit C₁-C₄-Alkyl oder C₁-C₄-Alkoxy substituiert sind. Y bedeutet einen linearen oder verzweigten Alkylenrest oder Alkenylenrest mit 2 bis 20, bevorzugt 2 bis 12 C-Atomen, der unsubstituiert oder mit C₁-C₆-Alkoxy, C₅- oder C₆-Cycloalkyl, C₅- oder C₆-Cycloalkoxy, Phenyl, Phenyloxy, Benzyl oder Benzyloxy substituiert ist, und die cyclischen Substituenten gegebenenfalls mit C₁-C₆-Alkyl oder C₁-C₆-Alkoxy substituiert sind. Die Gruppen R₁R₂X- beziehungsweise R₁R₂X'- sind im Alkylenrest oder Alkenylenrest in α,β-, α,γ- oder α,δ-Stellung gebunden, bevorzugt in α,β-Stellung oder α,γ-Stellung.

Einige Beispiele für Verbindungen der Formel I sind: 1,2-Diphenylphosphino- oder -arsinoethan, 1,2-Diphenylaminoethan, 1-Diphenylarnino-2-diphenylphosphinoethan, 1,2- oder 1,3-Diphenylphosphinopropan, 1,2-, 1,3-, 2,3- oder 1,4-Diphenylphosphinobutan, 1,2-Di(p-methylphenyl)phosphinoethan, 1,2-Di(p-methoxyphenyl)phosphinoethan, 1,2-Di[(phenyl-methyl)phosphino]ethan, 1,2-Diphenylphosphino-1-phenyl-ethan, 1,2-Diphenyl-1,2-diphenylphosphino-ethan, 1,2-, 1,3-, 1,4-, 2,3-, 3,4-, 2,4- oder 2,5-Diphenylphosphinohexan, 1,2-Diphenylphosphonitoethan, 1,2-Diphenylphosphinoethen, 1,2- oder 1,3-Diphenylphosphinopropen, 1,2-, 1,3-, 2,3- oder 1,4-Diphenylphosphinobut-1,2- oder -2,3-en.

In einer weiteren bevorzugten Ausführungsform entsprechen die zweizähnigen Liganden der Formel II, 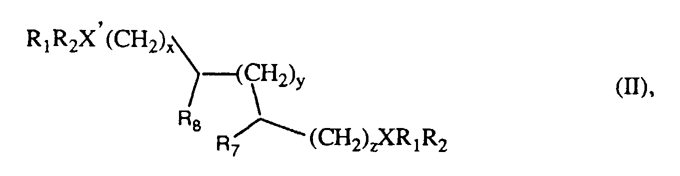 worin X und X' unabhängig voneinander für N, P, As, Sb oder -OP stehen, wobei P und -OP bevorzugt sind. X und X' sind bevorzugt gleich. Besonders bevorzugt stellen X und X' P dar. R₁ und R₂ können unabhängig voneinander C₁-C₁₂-, bevorzugt C₁-C₆-Alkyl, C₅-oder C₆-Cycloalkyl, Phenyl oder Benzyl sein, die unsubstituiert oder mit C₁-C₄-Alkyl oder C₁-C₄-Alkoxy substituiert sind. In der Formel II stehen x, y und z unabhängig voneinander für 0 oder 1, wobei die Summe von x+y+z 0, 1 oder 2, bevorzugt 0 oder 1 beträgt. R₇ und R₈ bilden zusammen mit dem Rest, an den sie gebunden sind, einen 5- oder 6-gliedrigen cycloaliphatischen Rest oder einen 5- oder 6-gliedrigen cycloheteroaliphatischen Rest mit ein oder zwei gleichen oder verschiedenen Heteroatomen aus der Gruppe O, S und NR₉, die unsubstituiert oder mit C₁-C₆-Alkyl oder C₁-C₆-Alkoxy oder Halogen wie zum Beispiel F, Cl und Br substituiert sind. R₉ stellt H, C₁-C₆-Alkyl, C₁-C₈-Acyl, oder unsubstituiertes oder mit C₁-C₆-Alkyl oder C₁-C₆-Alkoxy substituiertes Phenyl oder Benzyl dar. Wenn y für 0 steht, können R₇ und R₈ zusammen mit dem Rest, an den sie gebunden sind, einen 6-gliedrigen aromatischen oder heteroaromatischen Rest oder einen 5-gliedrigen heteroaromatischen Rest bilden, die unsubstiuiert oder mit C₁-C₆-Alkyl oder C₁-C₆-Alkoxy oder Halogen wie zum Beispiel F, Cl und Br substituiert sind. Die cycloaliphatischen Reste oder cycloheteroaliphatischen Reste können mono- oder polycyclisch, bevorzugt bicyclisch sein. Die heteroaromatischen Reste können O, S und N als Heteroatome enthalten.

Beispiele für R₇ und R₈ zur Bildung von gegebenenfalls wie zuvor definiert substituierten 5- oder 6-gliedrigen cycloaliphatischen Ringen sind 1,2-Ethylen, 1,2- oder 1,3-Propylen, 1-Phenyl-1,2-ethylen, 2-Phenyl-1,2- oder 1,3-Propylen, Cyclopent-3,4-en-1,5-diyl, Cyclopentan-1,5-diyl, Cyclohexan-3,6-diyl, Cyclohex-4,5-en-3,6-diyl, 1,4-Butylen, 2-Phenyl-1,4-butylen, 1,4-Pentylen, 3-Ethyl- 1,4-butylen, 1-Methylen-phen-2-yl, 1,2-Dimethylenbenzol.

Beispiele für R₇ und R₈ zur Bildung von gegebenenfalls wie zuvor definiert substituierten 5- oder 6-gliedrigen cycloheteroaliphatischen Ringen sind -CH₂O-, -CH₂OCH₂-, -OCH₂CH₂-, -CH₂OCH₂CH₂-, -CH₂OCH₂O-, -OCH₂CH₂O-, -OCR₁₀R₁₁O-, worin R₁₀ und R₁₁ unabhängig voneinander für H, C₁-C₆-Alkyl, unsubstituiertes oder mit C₁-C₆-Alkyl oder C₁-C₆-Alkoxy substituiertes Phenyl oder Benzyl darstellen, -CH₂NR₉-, -CH₂NR₉CH₂-, -NR₉CH₂CH₂-, -CH₂R₉NCH₂CH₂-, -CH₂NR₉CH₂NR₉-, -NR₉CH₂CH₂NR₉-, -NR₉CH₂NR₉-, worin R₉ H, C₁-C₆-Alkyl, C₁-C₈-Acyl, oder unsubstituiertes oder mit C₁-C₆-Alkyl oder C₁-C₆-Alkoxy substituiertes Phenyl oder Benzyl darstellt.

Beispiele für R₉ als Alkyl sind Methyl, Ethyl n- oder i-Propyl, n-, i- oder t-Butyl, Pentyl und Hexyl, als Acyl Formyl, Acetyl, Propionyl, Butanoyl, Benzoyl, Methylbenzoyl, Phenylacetyl, C₁-C₆-Alkoxy- oder C₅- oder C₆-Cycloalkoxy- oder Phenyloxy- oder Benzyloxycarbonyl.

Beispiele für R₇ und R₈ zur Bildung von gegebenenfalls wie zuvor definiert substituierten 5- oder 6-gliedrigen heteroaromatischen Ringen oder 6-gliedrigen aromatischen Ring sind 1,2-Phenylen, 2,3-Naphthylen, Pyrrol-3,4-diyl, Furan-3,4-diyl, 2,3- oder 3,4-Pyridin-diyl.

Einige Beispiele für die Liganden der Formel II sind 1,2-Diphenylphosphino- oder 1,2-Diphenylphosphinomethyl oder 1-Diphenylphosphino-2-diphenylphosphinomethyl- oder 1-Diphenylphosphino-3-diphenylphosphino-cyclopentan, -cyclohexan, -benzol, -indan; 2,3-Diphenylphoshinonaphthalin, Cyclohexan-1,2-bis(diphenylphosphonit), 1,2-Bis(dimethylaminomethyl)cyclohexan, 1-Dimethylaminomethyl-2-diphenylphosphinomethylcyclohexan, 1,2-Diphenylphoshinonorbornan oder -norbornen, 1,2-Diphenylphoshino-[2,2,2]-bicyclooctan oder -octen, 3,4-Diphenylphosphino- oder 3,4-Diphenylphosphinomethyl oder 3-Diphenylphosphino-4-diphenylphosphinomethyl- oder 2-Diphenylphosphino-4-diphenylphosphino- oder 2-Diphenylphosphino-4-diphenylphosphinomethyl-tetrahydrofuran, -tetrahydrothiophen oder -pyrrolidin, oder -N-methyl-, -N-butyl-, -N-benzyl-, -N-acetyl-, -N-benzoyl-, -N-phenoxycarbonyl- oder -N-t-butyloxycarbonyl-pyrrolidin, 2,3- oder 3,4-Diphenylphosphino- oder -Diphenylphosphinomethyl-piperazin, 2,3-Diphenylphosphino- oder -Diphenylphosphinomethyl-morpholin oder -dioxan, 4-Methyl- oder 4-Phenyl-1,2-Diphenylphosphino- oder 1,2-Diphenylphosphinomethyl oder 1-Diphenylphosphino-2-diphenylphosphinomethyl-cyclohexan, 4,5-Diphenylphosphino- oder 4,5-Diphenylphosphinomethyl oder 4-Diphenylphosphino-5-diphenylphosphinomethyl- 1,3-dioxolan oder -2-methyl, -2-butyl-, -2-phenyl-, -2-benzyl-, -2,2-dimethyl-, -2,2-diphenyl-, -2-methyl-2-phenyl-1,3-dioxolan.

Bei den zweizähnigen Liganden kann es sich auch um in den o,o'-Stellungen mit gleichen oder verschiedenen Gruppen -XR₁R₂ substituiertes Biphenylen oder Naphthylen handeln, worin die X unabhängig voneinander für N, P, As, Sb oder -OP stehen, wobei P und -OP bevorzugt sind. R₁ und R₂ können unabhängig voneinander C₁-C₁₂-, bevorzugt C₁-C₆-Alkyl, C₅- oder C₆-Cycloalkyl, Phenyl oder Benzyl sein, die unsubstituiert oder mit C₁-C₄-Alkyl oder C₁-C₄-Alkoxy substituiert sind. Die Biphenyl- beziehungsweise Naphthylreste können weitere Substituenten enthalten, zum Beispiel C₁-C₄-Alkyl, C₁-C₄-Alkoxy, F oder Cl. Einige Beispiele für solche Liganden sind 2,2'-Diphenylphosphino- oder -Dicyclohexylphosphinobiphenyl oder -binaphthyl.

In einer weiteren Ausführungsform kann es sich bei den zweizähnigen Liganden auch um Ferrocene der Formel III und IIIa handeln, 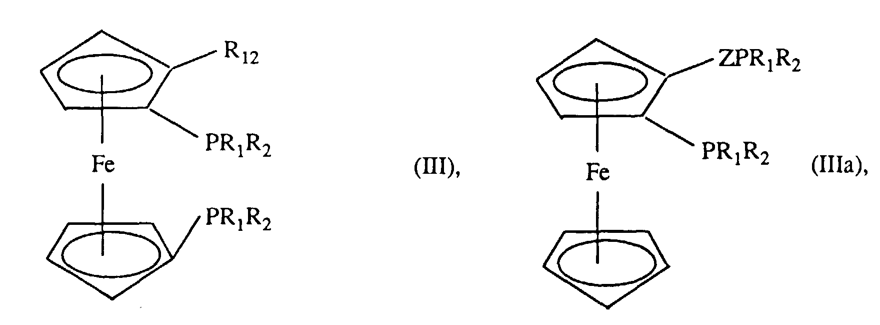 worin R₁ und R₂ unabhängig voneinander C₁-C₁₂-, bevorzugt C₁-C₆-Alkyl, C₅- oder C₆- Cycloalkyl, Phenyl oder Benzyl darstellen, die unsubstituiert oder mit C₁-C₄-Alkyl oder C₁-C₄-Alkoxy substituiert sind, R₁₂ für H, C₁-C₆-Alkyl, C₁-C₆-Alkenyl, Tri(C₁-C₆-Alkyl)silyl, Phenyl, Benzyl, α-Hydroxy- oder α-[Di(C₁-C₆-Alkyl)amino]-C₁-C₆-alkyl steht, und Z Methylen oder C₁-C₈-Alkyliden bedeutet. Beispiele für R₁₂ sind H, Methyl, Ethyl, 1-Vinyl, Allyl, Trimethylsilyl, Hydroxymethyl, 1-Hydroxy-eth-1-yl, Aminomethyl und 1-Dimethylamino-eth-1-yl. Beispiele für Z sind neben Methylen Ethyliden, 1,1- oder 2,2-Propyliden und Benzyliden.

In einer anderen Ausführungsform kann es sich bei den zweizähnigen Liganden um unsubstituiertes oder mit C₁-C₄-Alkyl, C₁-C₄-Alkoxy, F oder Cl substituiertes α,α'-Bipyridyl handeln.

In einer weiteren Ausführungsform kann es sich um Pyridin handeln, das in α-Stellung mit R₁₃N=CH- oder R₁₃N=CH-CH₂- substituiert ist, worin R₁₃ H, C₁-C₆-Alkyl, Phenyl oder Benzyl bedeutet. Ein Beispiel ist α-(N-Methyliminomethyl)pyridin.

Geeignete zweizähnige Liganden sind auch Bisoxazolidine der Formel IV, 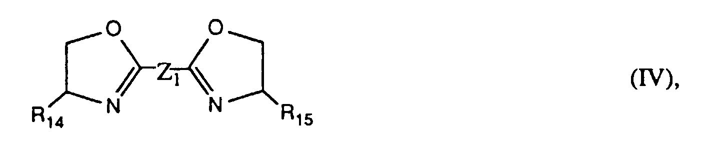 worin R₁₄ und R₁₅ gleich oder verschieden sind und H, lineares oder verzweigtes C₁-C₆-Alkyl, Phenyl, (C₁-C₆-Alkyl)phenyl, Benzyl oder (C₁-C₆-Alkyl)benzyl darstellen, und Z₁ für eine direkte Bindung, Methylen, C₂-C₈-Alkyliden, Ethylen, 1,2-Propylen oder 1,2-Phenylen steht. Beispiele für Alkyliden sind zuvor genannt worden.

Geeignete Bisoxazolidine sind auch solche der Formel V 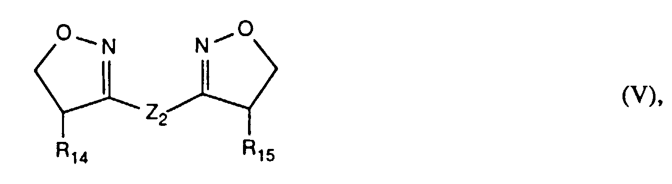 worin R₁₄ und R₁₅ gleich oder verschieden sind und H, lineares oder verzweigtes C₁-C₆-Alkyl, Phenyl, (C₁-C₆-Alkyl)phenyl, Benzyl oder (C₁-C₆-Alkyl)benzyl darstellen, und Z₂ für eine direkte Bindung, Methylen, 1,2- oder 1,3-Phenylen, Pyridin-2,6-diyl, Ethylen, 1,2-oder 1,3-Propylen oder 1,2-, 1,3-, 1,4- oder 2,3-Butylen oder C₂-C₈-Alkyliden steht.

Die Metallkomplexsalze können verschiedene einzähnige und/ oder mehrzähnige Liganden enthalten. Bevorzugte Kombinationen sind zwei verschiedene stark koordinierende zweizähnige Liganden, ein zweizähniger stark koordinierender Liganden und ein einzähniger stark koordinierender Ligand, ein zweizähniger stark koordinierender Ligand und zwei einzähnige schwach oder stark koordinierende Liganden, zwei verschiedene dreizähnige stark koordinierende Liganden oder ein dreizähniger stark koordinierender Ligand und drei einzähnige schwach oder stark koordinierende Liganden.

Die mehrzähnigen Liganden bilden mit dem Metallkation fünf- bis siebengliedrige Ringe, wobei fünf- und sechsgliedrige Ringe bevorzugt sind.

Die Metallkomplexsalze können nukleophile (koordinierende) Anionen enthalten. Beispiele für Halogenid sind besonders Chlorid, Bromid und Iodid. Beispiele für Pseudohalogen sind Cyanid und Cyanat. Das Alkoholat kann linear oder verzweigt sein und enthält bevorzugt 1 bis 4 C-Atome. Einige Beispiele sind Methylat, Ethylat, n- oder i-Propylat und n-, i- oder t-Butylat. Beispiele für Alkylphenolate sind Methyl-, Ethyl-, n- oder i-Propyl, n-, i- oder t-Butyl-, Dimethyl-, t-Butyl-methyl- und Di-t-butylphenolat. Beispiele für sekundäres Amido, das bevorzugt 2 bis 8 C-Atome enthält, sind Dimethyl-, Diethyl-, Di-n- oder -i-propyl-, Di-n-, -i- oder -t-butyl-, Methyl-ethyl-, Dicyclohexyl-, pyrrolidin-N-yl-, Piperazin-N-yl- und Morpholin-N-yl-amido. Beispiele für Silylamido sind Bis-(trimethylsilyl)- und Bis-(triethylsilyl)-amido. Beispiele für mit Alkyl substituiertes Cyclopentadienyl sind Methyl-, Dimethyl-, Pentamethyl-, Ethyl-, Propyl- und Butylcyclopentadienyl. Bevorzugt sind die Halogenide, besonders Chlorid, Bromid und Iodid.

Die Metallkomplexsalze enthalten nicht-nukleophile Anionen. Bei den Anionen von Sauerstoffsäuren kann es sich zum Beispiel um Sulfat, Phosphat, Perchlorat, Perbromat, Periodat, Antimonat, Arsenat, Nitrat, Carbonat, das Anion einer C₁-C₈-Carbonsäure wie zum Beispiel Formiat, Acetat, Propionat, Butyrat, Benzoat, Phenylacetat, Mono-, Di- oder Trichlor- oder -fluoracetat, Sulfonate wie zum Beispiel Methylsulfonat, Ethylsulfonat, Propylsulfonat, Butylsulfonat, Trifluormethylsulfonat (Triflat), Phenylsulfonat, Benzylsulfonat, Tosylat, Mesylat oder Brosylat, und Phosphonate wie zum Beispiel Methylphosphonat, Ethylphosphonat, Propylphoshonat, Butylphosphonat, Phenylphosphonat, p-Methylphenylphosphonat und Benzylphosphonat handeln. Bevorzugte Anionen sind Tetrafluorborat , Hexafluorphosphat, Perchlorat und Triflat.

In einer bevorzugten Ausführungsform des erfindungsgemässen Verfahrens entsprechen die Metallkomplexsalze der Formel VI

[(Q)ₘ(M⁺ⁿ)(S)ₒ(L⁻¹)_{q}]^{+(n-q)}(A^{-r})_{(n-q)/r} (VI),

worin Q gleiche oder verschiedene stark koordinierende Liganden bedeutet, m für 0 steht oder Q ein einzähniger Ligand und m eine Zahl von 1 bis 8, Q ein zweizähniger Ligand und m eine Zahl von 1 bis 4, Q ein dreizähniger Ligand und m die Zahlen 1 oder 2 oder Q ein vierzähniger Ligand und m die Zahlen 1 oder 2 sind, wobei die zwei-, drei- und vierzähnigen Liganden ein oder mehrere gleiche oder verschiedene Phosphin-, Phosphonit-, Arsin-, oder Stibingruppen oder primäre, sekundäre und/oder tertiäre Amingruppen und/oder Imingruppen als komplexbildende Gruppen enthalten, und wobei diese Liganden mit dem Metallkation M⁺ⁿ einen 5- bis 7-gliedrigen, bevorzugt einen 5- oder 6-gliedrigen Ring bilden; M ein Metall aus der zweiten bis fünften Hauptgruppe, der ersten bis achten Nebengruppe oder der Lanthaniden im Periodensystem der Elemente darstellt und n eine Zahl von 1 bis 4 ist; S ein schwach koordinierender Ligand aus der Gruppe Nitrile, aliphatische oder aromatische Alkohole, Ketone, Aldehyde, Carbonsäureester, Ether, Tris(trifluormethyl)phosphit, und Wasser bedeutet und o für 0 oder eine Zahl von 1 bis 6 steht; L Halogenid, Pseudohalogenid, C₁-C₈-Alkoholat oder unsubstituiertes oder mit C₁-C₄-Alkyl substituiertes Phenolat, sekundäres Amid mit 2 bis 12 C-Atomen, Bis-[(Tri-C₁-C₆-Alkyl)silyl]amid oder unsubstituiertes oder mit C₁-C₄-Alkyl substituiertes Cyclopentadienyl bedeutet und q für 0 oder eine Zahl von 1 bis 4 steht; A ein Anion einer Sauerstoffsäure, BF₄, PF₆, AsF₆ oder SbF₆ darstellt und r für eine Zahl von 1 bis 3 steht, wobei die Summe von (m x Zähnigkeit des Liganden Q) + o + q eine ganze Zahl von 2 bis 8 ist und die Summe von (m x Zähnigkeit des Liganden Q) + o mindestens 2 ist.

Für Q, M, S, L und A gelten die voranstehenden Bevorzugungen und beispielhaften Aufzählungen. In der Formel VI stehen r bevorzugt für 1, q bevorzugt für 0, 1 oder 2, o bevorzugt für 0, 1, 2 oder 3, n bevorzugt für 1, 2 oder 3 und m für 0 oder eine Zahl von 1 bis 6 und besonders bevorzugt eine Zahl von 1 bis 4 bei einzähnigen Liganden, eine Zahl von 1 bis 3, besonders bevorzugt die Zahlen 1 oder 2 bei zweizähnigen Liganden, und bevorzugt 1 bei drei und vierzähnigen Liganden. Wenn m 0 ist, steht o bevorzugt für eine Zahl von 2 bis 6. Ferner ist bevorzugt, dass M ein solvatisiertes Kation ist, besonders wenn m gleich 0 ist und der Ligand S gleichzeitig im erfindungsgemässen Verfahren als Lösungsmittel verwendet wird. Bevorzugte Kombinationen von Liganden Q und S sind: Q gleich zweizähniger Ligand, m gleich 1 oder 2 und o gleich 2; Q gleich dreizähniger Ligand, m gleich 1 und o gleich 3.

Die Metallkomplexsalze können beim erfindungsgemässen Verfahren als solche eingesetzt oder vor der Reaktion in situ hergestellt werden, besonders wenn der Ligand S gleichzeitig als Lösungsmittel dient.

Geeignete inerte Lösungsmittel sind zum Beispiel polare und bevorzugt aprotische Lösungsmittel, die alleine oder in Mischungen aus mindestens zwei Lösungsmitteln verwendet werden können. Beispiele sind: Ether (Dibutylether, Tetrahydrofuran, Dioxan, Ethylenglykolmonomethyl- oder -dimethylether, Ethylenglykolmonoethyl- oder -diethylether, Diethylenglykoldiethylether, Triethylenglykoldimethylether), halogenierte Kohlenwasserstoffe (Methylenchlorid, Chloroform, 1,2-Dichlorethan, 1,1,1-Trichlorethan, 1,1,2,2-Tetrachlorethan), Carbonsäureester und Lactone (Essigsäureethylester, Propionsäuremethylester, Benzoesäureethylester, 2-Methoxyethylacetat, γ-Butyrolacton, δ-Valerolacton, Pivalolacton), Carbonsäureamide und Lactame (N,N-Dimethylformamid, N,N-Diethylformamid, N,N-Dimethylacetamid, Tetramethylharnstoff, Hexamethylphosphorsäuretriamid, γ-Butyrolactam, ε-Caprolactam, N-Methylpyrrolidon, N-Acetylpyrrolidon, N-Methylcaprolactam), Sulfoxide (Dimethylsulfoxid), Sulfone (Dimethylsulfon, Diethylsulfon, Trimethylensulfon, Tetramethylensulfon), tertiäre Amine (N-Methylpiperidin, N-Methylmorpholin), aromatische Kohlenwasserstoffe wie zum Beispiel Benzol oder substituierte Benzole (Chlorbenzol, o-Dichlorbenzol, 1,2,4-Trichlorbenzol, Nitrobenzol, Toluol, Xylol) und Nitrile (Acetonitril, Propionitril, Benzonitril, Phenylacetonitril).

Bevorzugte Lösungsmittel sind halogenierte aliphatische Kohlenwasserstoffe, aromatische Kohlenwasserstoffe, Ether und Nitrile, zum Beispiel Methylenchlorid, Chloroform, Benzol, Toluol und Acetonitril.

Der geschützte Zucker und das Aglykon werden bevorzugt in equivalenten Mengen verwendet, wobei jedoch auch ein geringer Überschuss des Aglykons eingesetzt werden kann. Sofern ein Orthoester mitverwendet wird, wird dieser im allgemeinen im Überschuss eingesetzt, zum Beispiel in Mengen von 1,2 bis 10, bevorzugt 1,2 bis 5 und besonders bevorzugt 1,5 bis 3 Äquvialenten pro Äquivalent geschütztem Zucker.

Die Reaktionstemperatur kann bevorzugt -20 bis 250 °C, besonders bevorzugt 10 bis 200 °C und insbesondere bevorzugt 20 bis 150 °C betragen. Eine besonders bevorzugte Ausführungsform stellt die Mitverwendung eines Orthoesters dar, was den Vorteil bietet, dass das Verfahren bei Raumtemperatur durchgeführt werden kann.

Die Reaktionszeit kann von Minuten bis Tage reichen, was hauptsächlich von der Wahl des Katalysators, der Art der Reaktanden und den Reaktionsbedingungen abhängt.

Das erfindungsgemässe Verfahren kann in unterschiedlicher Weise durchgeführt werden. Zweckmässig wird das entstehende Reaktionswasser entweder chemisch gebunden (zum Beispiel mit Hilfe von Orthoestern) oder laufend aus dem Reaktionsgemisch entfernt, zum Beispiel durch Destillation oder durch Zugabe von Wasser adsorbierenden Mitteln wie zum Beispiel Silicagelen oder Molekularsieben, oder durch Zugabe von wasserbindenden, mindestens teilweise entwässerten anorganischen Salzen, zum Beispiel Na₂SO₄, MgSO₄, CaSO₄, MgCl₂ oder CaCl₂.

In einer Verfahrensvariante kann man so vorgehen, dass man den geschützten Zucker und den Katalysator in einem Lösungsmittel bei Raumtemperatur vorlegt, und dann das Aglykon und einen Orthoester zugibt. Das Gemisch wird dann bei Raumtemperatur bis zur Rückflusstemperatur der Lösungsmittel gerührt. In einer bevorzugten Ausführungsform verwendet man bei dieser Variante Orthoester von Alkoholen, die gleichzeitig als Aglykon eingesetzt werden.

In einer anderen Verfahrensvariante kann man so vorgehen, dass man den geschützten Zucker und das Aglykon in einem Lösungsmittel vorlegt und dann den Katalysator zugibt. Anschliessend kann man ein Molekularsieb oder zum Beispiel Magnesiumsulfat zugeben. Vorteilhafter ist es, das Wasser bindende Mittel wie zum Beispiel das Molekularsieb in einem Soxhlet in einer Hülse vorzulegen. Das Reaktionsgemisch wird dann am Rückfluss erhitzt bis die Reaktion beendet ist.

In einer weiteren Verfahrensvariante kann man so vorgehen, dass man den geschützten Zucker und das Aglykon in einem Lösungsmittel vorlegt und dann den Katalysator zugibt, wobei man eine Apparatur mit Wasserabscheider verwendet. Das Reaktionsgemisch wird dann am Rückfluss erhitzt und das Reaktionswasser azeotrop abdestilliert, bis die Reaktion beendet ist.

Das Reaktionsende kann durch spektroskopische oder chromatographische Verfahren ermittelt werden. Zur Isolierung des gewünschten Reaktionsprodukts lässt man das Reaktionsgemisch abkühlen und gewinnt das Produkt durch übliche Trennverfahren wie Destillation, Extraktion, Kristallisation oder chromatographische Verfahren.

Mit dem erfindungsgemässen Verfahren können überraschend anomere Hydroxylgruppen von geschützten Zuckern direkt glykolisiert werden, wobei man zudem sehr hohe Ausbeuten und Produktereinheiten erzielt. Zudem kann das Verhältnis von α- zu β-Form beeinflusst werden, so dass man in einzelnen Fällen sogar die eine oder andere Form in praktisch quantitativer Ausbeute erhält.

Das erfindungsgemässe Verfahren eignet sich zur Einführung von Schutzgruppen in der anomeren Hydroxylgruppe oder zur Synthese von Oligosacchariden, Glykolipiden und Glykopeptiden (siehe zum Beispiel H. Paulsen, Chem. Soc. Rev. 13, Seiten 15 bis 45 (1984) und R. R. Schmidt, Angew. Chem. 98, Seiten 213 ff (1986). Das Verfahren eignet sich auch zur Synthese von pharmazeutischen Wirkstoffen oder Agrochemikalien, zum Beispiel Naturstoffen wie Pheromonen oder Derivaten davon, was im nachfolgenden Anwendungsbeispiel beschrieben ist.

Die nachfolgenden Beispiele erläutern die Erfindung näher.

### A) Herstellungsbeispiele

### Beispiele 1 bis 24 (Orthoesterverfahren):

In einem Rundkolben werden 1 Equivalent geschützter Zucker (Glykosyldonor) und 0,5 Mol-% Katalysator (Beispiel 11: 5 Mol-%, Beispiel 21: 0,15 Mol-%) in 30 ml Methylenchlorid gelöst und dann mit 1 Equivalent Aglykon (Glykosylacceptor) versetzt. Dann rührt man das Gemisch bei Raumtemperatur (Beispiele 9, 11 und 12: 40 °C). Das Reaktionsende wird dünnschichtchromatographisch (DC-Fertigplatten, Merck, Kieselgel 60 F₂₅₄) ermittelt. Zur Isolierung der Verbindungen wird das Reaktionsgemisch eingedampft und an Kieselgel 60 (Korngrösse 0,04 bis 0,063 mm) chromatographiert). Das Verhältnis von α- zu β-Form wird mittels ¹H-NMR ermittelt. Weitere Angaben befinden sich in Tabelle 1.

Es werden die folgenden Katalysatoren verwendet:
A: [(1,1,1-Tri(methyl-phenylphosphinomethyl)ethan)Rh(III)(NCCH₃)₃](CF₃CO₂)₃
B: [(1,2-Diphenylphosphinoethan)Pd(II)(H₂O)₂](CF₃CO₂)₂
C: [(R(+)-2,2'-diphenylphosphinobinaphthyl)Pd(II)(H₂O)₂](CF₃CO₂)₂
D: [(S(-)-2,2'-diphenylphosphinobinaphthyl)Pd(II)(H₂O)₂](CF₃CO₂)₂
E: [(1,1,1-Tri(phenylphosphinomethyl)ethan)Ru(II)(NCCH₃)₃](CF₃CO₂)₂
F: [(1,1,1 Tri(phenylphosphinomethyl)ethan)Rh(III)(NCCH₃)₃](CF₃CO₂)₃

Folgende geschützte Zucker werden verwendet: 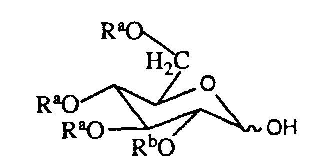
R^{a},R^{b} = Benzyl (1a)
R^{a},R^{b} = Acetyl (1b)
R^{a},R^{b} = Pivaloyl (1c)
R^{a} = Benzyl, R^{b} = Methyl (1d)
R^{a} = Benzyl, R^{b} = Dimethyl-t-butylsilyl (1e)
R^{a} = Benzyl, R^{b} = Acetyl (1f)
R^{a} = Benzyl, R^{b} = Trichloracetyl (1g)
R^{a} Benzyl, R^{b} Benzoyl (1h)
R^{a} Benzyl, R^{b} Pivaloyl (1i)

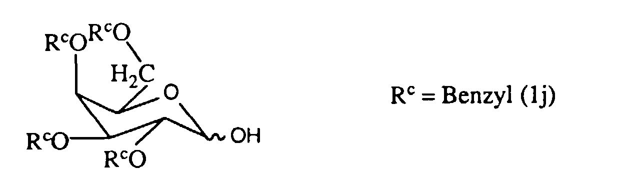

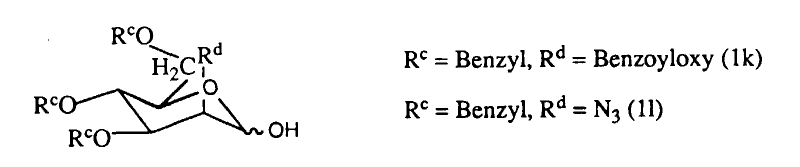

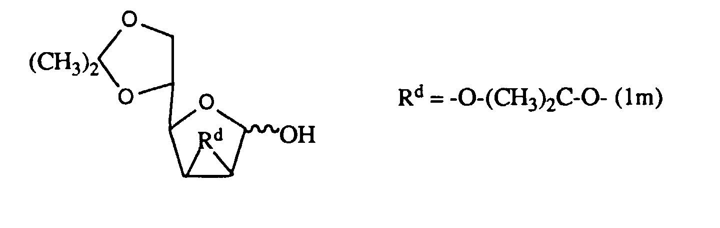

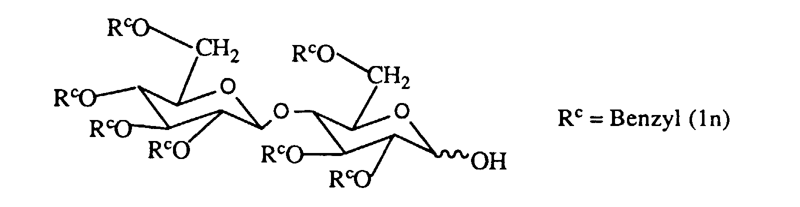

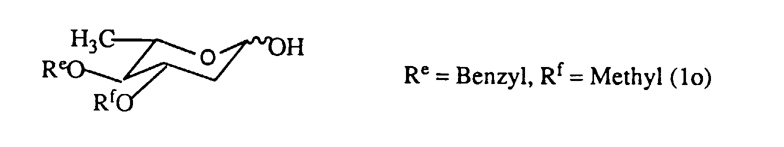

**Tabelle 1:**

| Beipiel Nr. | Zucker | Aglykon | Orthoester | Katalysator | Reaktionszeit (min.) | Ausbeute (%) | Verhältnis α:β-Form |
|---|---|---|---|---|---|---|---|
| 1 | 1a | CH₃OH | HC(OCH₃)₃ | A | 300 | 91 | 47:51 |
| 2 | 1a | " | " | B | " | 91 | 50:50 |
| 3 | 1a | " | " | C | 240 | 88 | 53:47 |
| 4 | 1a | " | " | D | 240 | 88 | 50:50 |
| 5 | 1a | " | " | E | 6 Tage | 24 | 48:52 |
| 6 | 1a | " | " | F | 10 | 92 | 59:41 |
| 7 | 1a | C₂H₅OH | HC(C₂H₅O)₃ | F | 10 | 85 | 50:50 |
| 8 | 1a | Allylalkohol | HC(Allyl-O)₃ | F | 5 | 96 | 62:38 |
| 9 | 1a | i-Propanol | HC(i-Propyl-O)₃ | F | 390 | 81 | 65:35 |
| 10 | 1a | Phenol | HC(C₆H₅O)₃ | F | 10 | 72 | 37:63 |
| 11 | 1b | CH₃OH | HC(CH₃O)₃ | F | 48 Stunden | 52 | 11:89 |
| 12 | 1c | " | " | F | 18 Stunden | 60 | 4:96 |
| 13 | 1d | " | " | F | 5 | 90 | 56:44 |
| 14 | 1e | " | " | F | 30 | 88 | 48:52 |
| 15 | 1f | " | " | F | 240 | 86 | 3:97 |
| 16 | 1g | " | " | F | 114 Stund. | 59 | 20:80 |
| 17 | 1h | " | " | F | 90 | 85 | 5:95 |
| 18 | 1i | " | " | F | 30 | 69 | 1:99 |
| 19 | 1j | " | " | F | 10 | 93 | 60:40 |
| 20 | 1k | " | " | F | 105 | 88 | 64:36 |
| 21 | 1l | Phenol | HC(C₆H₅O)₃ | F | 150 | 89 | 97:3 |
| 22 | 1m | CH₃OH | HC(CH₃O)₃ | F | 1 | 65 | 99:1 |
| 23 | 1n | " | " | F | 120 | 93 | 60:40 |
| 24 | 1o | i-Propanol | HC(i-PropylO)₃ | F | 1 | 70 | 50:50 |

### Beispiele 25-34, 35-39, 40-43, 44-56, 57-72, 73-85:

Es wird gemäss Beispiel 1 verfahren und Tetra-O-benzyl-D-glucopyranose als geschützter Zucker, Methanol als Aglykon und Orthoameisensäuremethylester verwendet. Die Reaktion wird bei Raumtemperatur durchgeführt. Die Ergebnisse sind in den Tabellen 2 bis 7 dargestellt Abkürzungen: Ph = Phenyl, Tf = Triflat. Der Index n bei den Komplexkationen bedeutet solvatisierte Metallkationen.

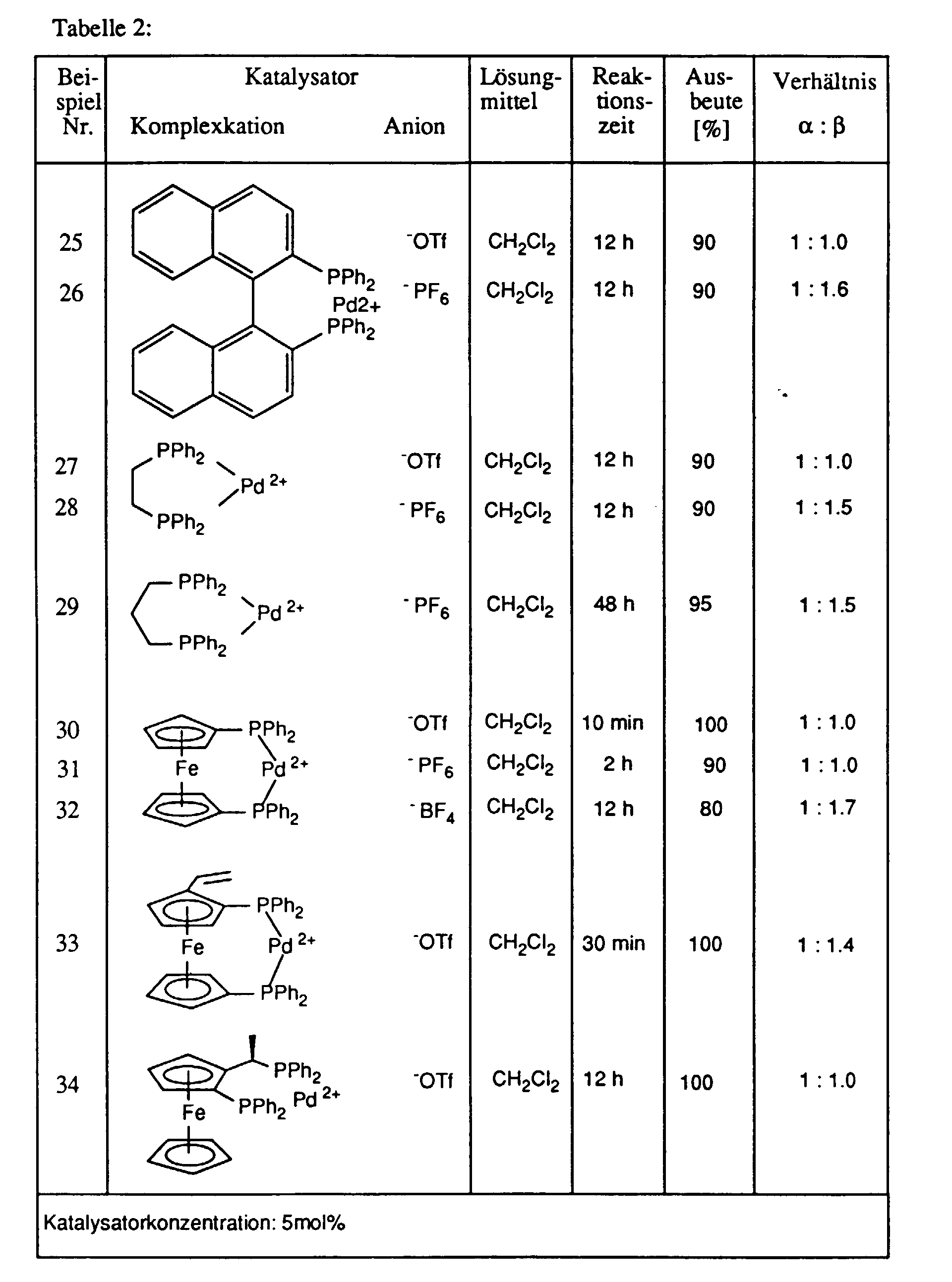

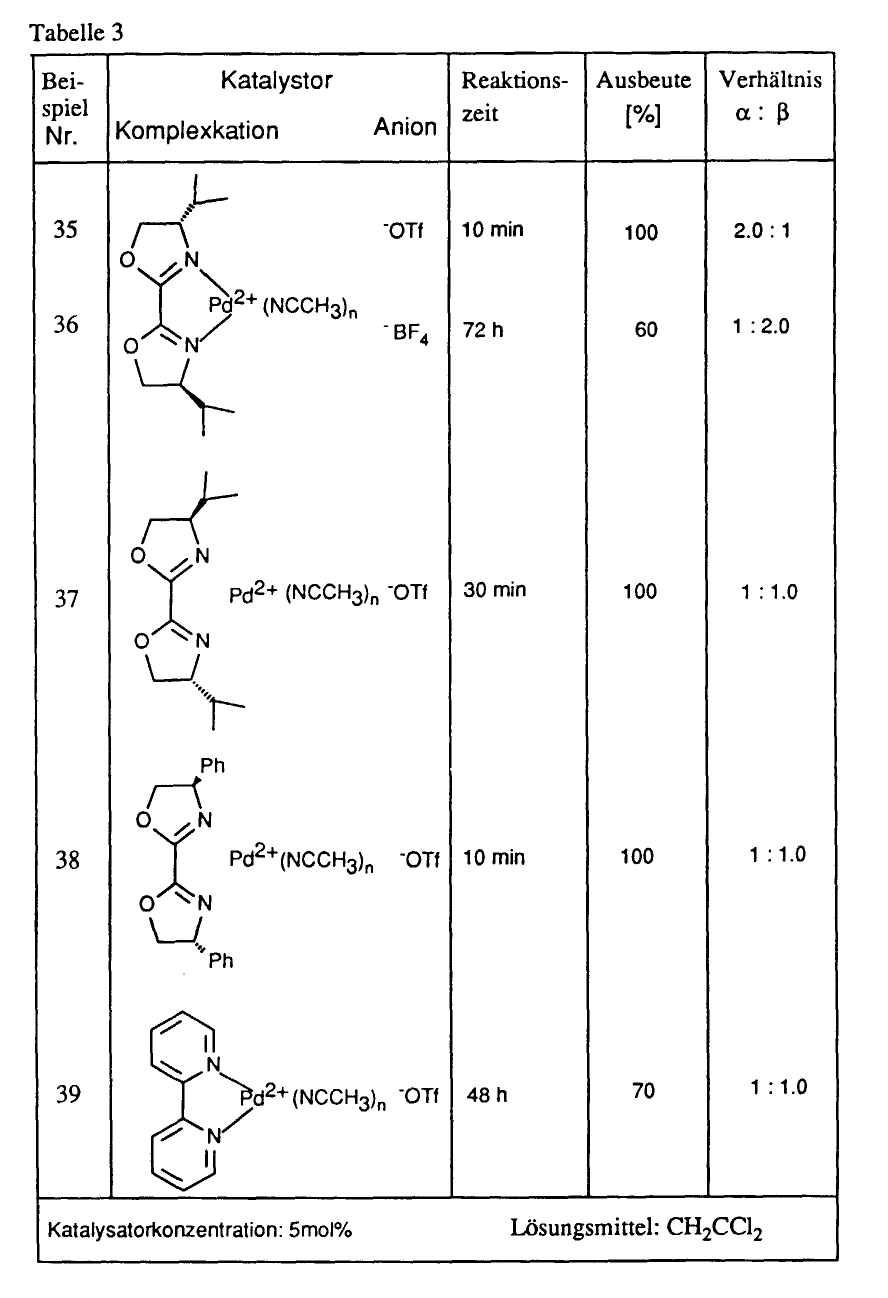

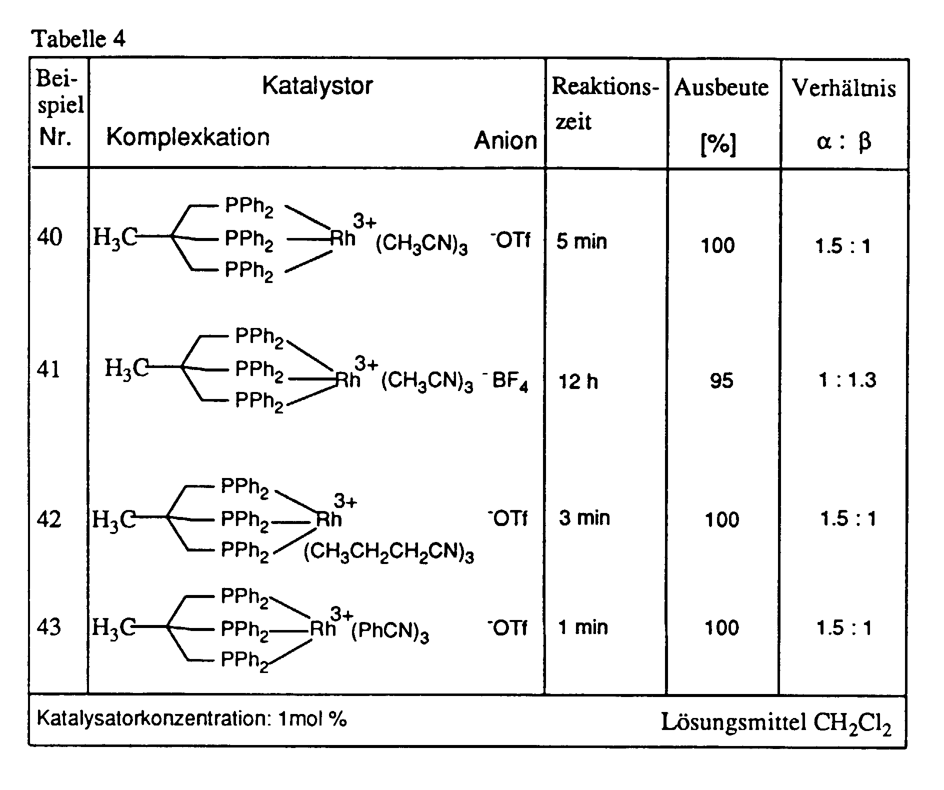

**Tabelle 5**

| Beispiel Nr. | Katalystor | | Lösungsmittel | Reaktionszeit | Ausbeute [%] | Verhältnis α: β |
|---|---|---|---|---|---|---|
| | Komplexkation | Anion | | | | |
| 44 | (C₆H₅CN)ₙ-Pt²⁺ | ⁻OTf | CH₂Cl₂ | 12 h | 100 | 1 : 1.0 |
| 45 | (C₆H₅CN)ₙ-Pt²⁺ | ⁻BF₄ | CH₂Cl₂ | 12 h | 60 | 1: 3.0 |
| 46 | (CH₃CN)ₙ-Cu⁺ | ⁻OTf | CH₂Cl₂ | 20 min | 100 | 1 : 1.0 |
| 47 | (CH₃CN)ₙ-Cu⁺ | ⁻BF₄ | CH₂Cl₂ | 72 h | 60 | 1 : 1.3 |
| 48 | (CH₃CN)ₙ-Ce³⁺ | ⁻OTf | CH₂Cl₂ | 1 h | 100 | 1 :1.0 |
| 49 | (CH₃CN)ₙ-Ce³⁺ | ⁻OTf | CH₂Cl₂/CH₃CN 10/3 | 12 h | 80 | 1:3.0 |
| 50 | (CH₃CN)ₙ-Ce³⁺ | ⁻BF₄ | CH₂Cl₂ | 1 h | 95 | 1 : 1.6 |
| 51 | (CH₃CN)ₙ-Ce³⁺ | ⁻BF₄ | CH₂Cl₂/CH₃CN 10/3 | 12 h | 40 | 1: 2.8 |
| 52 | (CH₃CN)ₙ-Mg²⁺ | ⁻OTf | CH₂Cl₂ | 12 h | 80 | 1 : 1.5 |
| 53 | (CH₃CN)ₙ-Mg²⁺ | ⁻OTf | CH₂Cl₂/CH₃CN 10/3 | 12 h | 50 | 1 : 2.0 |
| 54 | (CH₃CN)ₙ-Mg²⁺ | ⁻BF₄ | CH₂Cl₂ | 15 min | 100 | 1 : 1.7 |
| 55 | (CH₃CN)ₙ-Mg²⁺ | ⁻BF₄ | CH₂Cl₂/CH₃CN 10/3 | 12 h | 60 | 1 : 3.0 |
| 56 | (H₂O)-Mg²⁺ | ⁻ClO₄ | CH₂Cl₂ | 72 h | 100 | 1 : 1.3 |
| Katalysatorkonzentration: 5mol% | | | | | | |

**Tabelle 6**

| Beispiel Nr. | Katalystor | | Lösungsmittel | Reaktionszeit | Ausbeute [%] | Verhältnis α : β |
|---|---|---|---|---|---|---|
| | Komplexkation | Anion | | | | |
| 57 | ((CH₃CH₂)₂Oₙ)-Ag⁺ | ⁻OTf | CH₂Cl₂ | 75 min | 90* | 1 : 1.4 |
| 58 | ((CH₃CH₂)₂Oₙ)-Ag⁺ | ⁻BF₄ | CH₂Cl₂ | 5 h | 55* | 1 : 1.4 |
| 59 | ((CH₃CH₂)₂Oₙ)-Ag⁺ | ⁻PF₆ | CH₂Cl₂ | 5 h | 55* | 1 : 1.4 |
| 60 | (H₂O)-Ag⁺ | ⁻ClO₄ | CH₂Cl₂ | 5 h | 95* | 1 : 1.8 |
| 61 | (CH₃CN)ₙ-Rh³⁺ | ⁻OTf | CH₂Cl₂ | 5 min | 100 | 1 : 1.0 |
| 62 | (CH₃CN)ₙ-Rh³⁺ | ⁻OTf | CH₂Cl₂/CH₃CN 10/3 | 12 h | 90 | 1 : 2.9 |
| 63 | (CH₃CN)ₙ-Rh³⁺ | ⁻BF₄ | CH₂Cl₂ | 12 h | 80 | 1 : 1.2 |
| 64 | (CH₃CN)ₙ-Rh³⁺ | ⁻BF₄ | CH₂Cl₂/CH₃CN 10/3 | 12 h | 50 | 1 : 3.8 |
| 65 | ((CH₃CH₂)₂O)ₙ-Zn²⁺ | ⁻OTf | CH₂Cl₂ | 10 min | 100 | 1 : 1.0 |
| 66 | ((CH₃CH₂)₂O)ₙ-Zn²⁺ | ⁻OTf | CH₂Cl₂/CH₃CN 10/3 | 12 h | 100 | 1 : 3.6 |
| 67 | (CH₃CN)ₙ-Zn²⁺ | ⁻BF₄ | CH₂Cl₂ | 1 h | 100 | 1 : 1.9 |
| 68 | (CH₃CN)ₙ-Zn²⁺ | ⁻BF₄ | CH₂Cl₂/CH₃CN 10/3 | 12 h | 80 | 1 : 2.7 |
| 69 | ((CH₃CH₂)₂O)ₙ-Sn²⁺ | ⁻OTf | CH₂Cl₂ | 1 min | 100 | 1 : 1.0 |
| 70 | ((CH₃CH₂)₂O)ₙ-Sn²⁺ | ⁻OTf | CH₂Cl₂/CH₃CN 10/3 | 12 h | 100 | 1 : 2.5 |
| 71 | ((CH₃CH₂)₂O)ₙB³⁺ | ⁻F | CH₂Cl₂ | 90 min | 100 | 1 : 4.0 |
| 72 | ((CH₃CH₂)₂O)ₙB³⁺ | ⁻F | CH₂Cl₂/CH₃CN 10/3 | 4 h (-20°C) | 60 | 1 : 14.0 |
| Katalysatorkonzentration: 5 mol% | | | | | | |

| | | | | | | |
|---|---|---|---|---|---|---|
| *Reaktionsloesunge sind heterogen | | | | | | |

**Tabelle 7**

| Beispiel Nr. | Katalystor | | Lösungsmittel | Reaktionszeit | Ausbeute [%] | Verhältnis α : β |
|---|---|---|---|---|---|---|
| | Komplexkation | Anion | | | | |
| 73 | (CH₃CN)ₙ-Pd²⁺ | ⁻OTf | CH₂Cl₂ | 10 min | 100 | 1 : 1.0 |
| 74 | (CH₃CN)ₙ-Pd²⁺ | ⁻PF₆ | CH₂Cl₂ | 12 h | 100 | 1 : 1.3 |
| 75 | (CH₃CN)ₙ-Pd²⁺ | ⁻BF₄ | CH₂Cl₂ | 12 h | 60* | 1 : 1.3 |
| 76 | (CH₃CN)ₙ-Pd²⁺ | ⁻BF₄ | CH₃CN | 12 h | 95 | 1 : 4.0 |
| 77 | (CH₃CN)ₙ-Pd²⁺ | ⁻BF₄ | CH₂Cl₂/CH₃NO₂ 10/3 | 10 min | 100 | 1 : 1.2 |
| 78 | (CH₃CN)ₙ-Pd²⁺ | ⁻BF₄ | CH₂Cl₂/CH₃CN 10/1 | 20 h | 100 | 1 : 3.1 |
| 79 | (CH₃CN)ₙ-Pd²⁺ | ⁻BF₄ | CH₂Cl₂/CH₃CN 10/2 | 20 h | 100 | 1 : 4.2 |
| 80 | (CH₃CN)ₙ-Pd²⁺ | ⁻BF₄ | CH₂Cl₂/CH₃CN 10/3 | 20 h | 100 | 1 : 5.4 |
| 81 | (CH₃CN)ₙ-Pd²⁺ | ⁻BF₄ | CH₂Cl₂/CH₃CN 10/6 | 20 h | 80 | 1 : 5.0 |
| 82 | (CH₃CN)ₙ-Pd²⁺ | ⁻BF₄ | CH₂Cl₂/CH₃CN 10/10 | 20 h | 70 | 1 : 4.0 |
| 83 | (CH₃CN)ₙ-Pd²⁺ | ⁻BF₄ | CH₂Cl₂/CH₃CN 10/3 | 72 h | 70 | 1 : 4.0 |
| 84 | (CH₃CN)ₙ-Pd²⁺ | ⁻BF₄ | CH₂Cl₂/CH₃CN 10/10 | 72 h | 70 | 1 : 5.6 |
| 85 | (CH₃CN)ₙ-Pd²⁺ | ⁻OTf | CH₂Cl₂/CH₃CN 10/3 | 12h | 100 | 1 : 2.5 |
| Katalysatorkonzentration: 5mol% | | | | | | |
| Beispiel 83: i-Propanol/Tri-i-propanolformiat, | | | | | | |
| Beispiel 84: Allylalkohol/(CH₂=CHCH₂O)₃CH0 | | | | | | |

| | | | | | | |
|---|---|---|---|---|---|---|
| * Reakionsloesung heterogen! | | | | | | |

### Beispiele 86-103 (Soxhletverfahren):

In einem 50 ml Rundkolben mit Soxhlet, der eine Hülse mit einem aktivierten Molekularsieb (4 Angstöm) enthält, werden 1 Äquivalent des geschützten Zuckers (siehe Beispiel 1) und 1 Äquivalent des Aglykons in 30 ml Methylenchlorid mit 0,5 Mol-% (Beispiel 89: 2,5 Mol-%) Katalysator F versetzt. Danach erwärmt man bis auf Rückflusstemperatur (40 C°) und lässt bei dieser Temperatur reagieren. Nach dem Abkühlen wird das Lösungsmittel abgedampft und der Rückstand gemäss Beispiel 1 chromatographiert. Weitere Angaben befinden sich in Tabelle 8.

Es werden folgende Aglykone verwendet:
Benzylalkohol (2a), p-Methoxyphenol (2b), 8-(p-Methoxyphenyloxy)-octan-1-ol (2c), Cholesterol (2d), 3,7-Dimethyl-oct-6-en-1-ol (2e), 1-(p-Methoxyphenyloxy)-7-benzoyloxy-tetradecan-14-ol (2f), 2-Azido-dodecan-1-ol (2g), 2-Azido-3-benzyloxy-oct-4-en-1-ol (2h), und geschützte Zucker der Formel 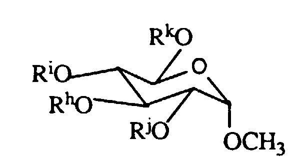
2i: R^{h}, Rⁱ und R^{j} = Benzyl, R^{k} = Wasserstoff.
2j: R^{h}, Rⁱ und R^{k} = Benzyl, R^{j} = Wasserstoff.
2k: R^{h}, R^{j} und R^{k} = Benzyl, Rⁱ = Wasserstoff.

**Tabelle 8:**

| Beispiel Nr. | Zucker | Aglykon | Reaktionzeit (Stunden) | Ausbeute (%) | Verhältnis α:β |
|---|---|---|---|---|---|
| 86 | 1a | 2a | 23 | 87 | 64:36 |
| 87 | 1a | 2b | 23 | 64 | 64:36 |
| 88 | 1a | 2c | 20 | 94 | 59:41 |
| 89 | 1a | 2d | 17 | 67 | 66:34 |
| 90 | 1a | 2e | 40 | 88 | 64:36 |
| 91 | 1a | 2i | 27 | 81 | 74:26 |
| 92 | 1h | 2c | 16 | 96 | β-Form |
| 93 | 1h | 2f | 24 | 95 | β-Form |
| 94 | 1h | 2i | 15,5 | 75 | β-Form |
| 95 | 1f | 2c | 19 | 71 | β-Form |
| 96 | 1f | 2i | 21,5 | 56 | β-Form |
| 97 | 1j | 2c | 21,5 | 71 | 60:40 |
| 98 | 1k | 2c | 260 | 20 | 60:40 |
| 99 | 1n | 2c | 20 | 46 | 50:50 |
| 100 | 1h | 2j | 20 | 78 | β-Form |
| 101 | 1h | 2k | 48 | 27 | β-Form |
| 102 | 1h | 2g | 24 | 90 | β-Form |
| 103 | 1h | 2h | 24 | 43 | β-Form |

### Beispiele 104-108 (Wasserabscheideverfahren):

In einem 50 ml Rundkolben mit Wasserabscheider werden 1 Äquivalent des geschützten Zuckers und ein Äquivalent des Aglykons in 25 ml Benzol (Beispiel 105: CH₂Cl₂) gelöst und mit 0,5 Mol-% Katalysator F versetzt. Dann wird das Gemisch bis zum Rückfluss auf 80 °C (Beispiel 105: 40 °C) erhitzt und bei dieser Temperatur gerührt. Zur Isolierung der Reaktionsprodukte wird das Lösungsmittel abdestilliert und der Rückstand gemäss Beispiel 1 chromatographiert. Weitere Angaben befinden sich in Tabelle 9.

**Tabelle 9:**

| Beispiel Nr. | Zucker | Aglykon | Reaktionszeit (Stunden) | Ausbeute (%) | Verhältnis α:β |
|---|---|---|---|---|---|
| 104 | 1a | 2l | 40 | 50 | 65:35 |
| 105 | 1a | 2m | 22 | 30 | 64:36 |
| 106 | 1a | 2c | 44 | 69 | 50:50 |
| 107 | 1i | 2c | 134 | 72 | 30:70 |
| 108 | 1n | 2i | 38 | 41 | 60:40 |
| 2l = 1-(p-Methoxyphenyloxy)-5-hydroxy-hexan | | | | | |

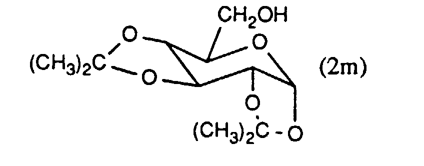

### Beispiele 109-111: Verwendung wasserentziehender Reagenzien.

In einem 50 ml Rundkolben werden 1 Äquivalent des geschützten Zuckers und 1 Äquivalent des Aglykons in 25 ml CH₂Cl₂ gelöst, mit 1 g MgSO₄ versetzt und 3 Mol% Fe(ClO₄)₃ als Katalysator zugegeben. Dann wird das Gemisch bis zum Rückfluss erhitzt und 1 Stunde bei dieser Temperatur gerührt. Zur Isolierung der Reaktionsprodukte wird das Lösungsmittel am Rotationsverdampfer entfernt und der Rückstand gemäss Beispiel 1 chromatographiert. Weitere Angaben befinden sich in Tabelle 10.

**TABELLE 10**

| Beispiel Nr. | Zucker | Aglykon | Ausbeute (%) | Verhältnis α : β |
|---|---|---|---|---|
| 109 | 1a | 2c | 95 | 62 : 38 |
| 110 | 1a | 2n | 76 | 50 : 50 |
| 111 | 1h | 2c | 94 | β-Form |
| 2n = Cyclohexanol | | | | |

B) Anwendungsbeispiel: Synthese der bei Kirschfliegen die Eiablage hemmenden Verbindung (13).

### Beispiel B1:

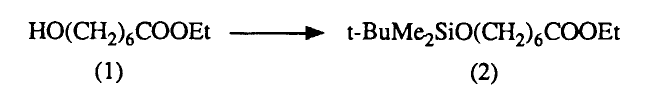

16,42 g (94,36 mMol) (1) in 180 ml Dimethylformamid (DMF) werden bei Raumtemperatur (RT) mit 14,95 g (99,1 mMol) t-Butyldimethylchlorsilan und 13,49 g (198,2 mMol) Imidazol versetzt und bei RT über Nacht gerührt. Danach wird das Reaktionsgemisch in 300 ml CH₂Cl₂ aufgenommen, je einmal mit Wasser und konzentrierter wässriger NaCl (Sole) gewaschen und dann über Na₂SO₄ getrocknet. Das Lösungsmittel wird am Rotationsverdampfer (ROT) abgedampft und der Rückstand (27,4 g) ohne weitere Reinigung in Beispiel B2 eingesetzt.
Abkürzungen: Et = Ethyl, Me = Methyl, Bu = Butyl, Bn = Benzyl, Bz = Benzoyl.

### Beispiel B2:

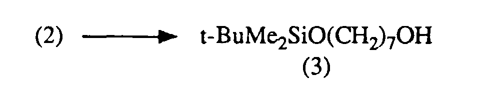

Der Rückstand gemäss Beispiel B1 wird in 60 ml Diethylether (Ether) gelöst und während 30 Minuten zu einer Lösung von 3,59 g (94,36 mMol) LiAlH₄ in 150 ml Ether getropft Anschliessend rührt man noch eine Stunde am Rückfluss. Danach wird das Reaktionsgemisch bei 0 °C nacheinander mit 10,8 ml H₂O, 10,8 ml NaOH und 32,4 ml H₂O versetzt, 10 Minuten gerührt, über Diatomeenerde filtriert und das Filtrat mit MgSO₄ getrocknet. Das Lösungsmittel wird am ROT entfernt und der Rückstand an Kieselgel (Hexan/Ether/3:1) chromatographiert. Man erhält 20,94 g (3); ¹H-NMR (250 MHz, CDCl₃): 3,55 (t, J=7, -CH₂-OSi).

### Beispiel B3:

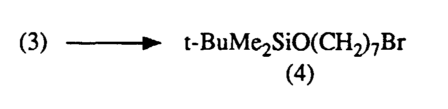

Zu 3,76 g (15,25 mMol) (3) in 24 ml Benzol werden bei RT tropfenweise 2,4 ml 1-Brom-2-N,N-trimethyl-propenylamin zugegeben. Anschliessend rührt man 20 Minuten bei RT und entfernt danach das Lösungsmittel am ROT entfernt und der Rückstand an Kieselgel (Hexan/Ether/19:1) chromatographiert. Man erhält 4,53 g (4); ¹H-NMR (250 MHz, CDCl₃): 3,55 (t, J=7, -CH₂Br).

### Beispiel B4:

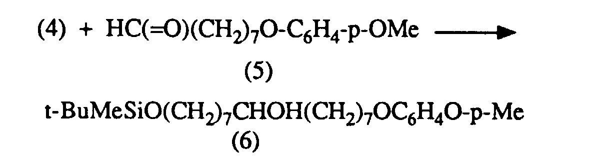

4,46 g (14,41 mMol) (4) in 15 ml Tetrahydrofuran (THF) werden bei RT mit 350 mg Magnesium umgesetzt. Nach dem Abkühlen des Reaktionsgemisches 3,61 g (14,41 mMol) (5) in 10 ml THF unter Eiskühlung zugetropft. Man lässt auf Raumtemperatur erwärmen und rührt eine Stunde. Danach gibt man 15 ml 2N H₂SO₄ und 50 ml Ether zu und trennt die organische Phase ab. Man wäscht mit Wasser und Sole, trocknet über Na₂SO₄ und entfernt das Lösungsmittel am ROT. Der Rückstand wird an Kieselgel (Pentan/Essigsäureethylester/8:1) chromatographiert. Man erhält 5,82 g (6); ¹H-NMR (250 MHz, CDCl₃): 3,90 (t, J=7, -CH₂O-).

### Beispiel B5:

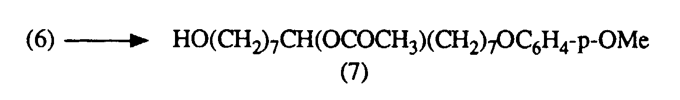

2,51 g (5,24 mMol) (6), 1,7 ml Essigsäureanhydrid und 2,8 ml Pyridin in 5 ml CH₂Cl₂ gelöst werden über Nacht bei RT gerührt. Dann wird das Reaktionsgemisch mit Essigsäureethylester verdünnt, je einmal mit 1N HCl, H₂O, gesättigter wässriger NaHCO₃ und Sole gewaschen und danach mit Na₂SO₄ getrocknet. Man entfernt dann das Lösungsmittel am ROT, löst dann den Rückstand in THF, versetzt mit (n-Butyl)₄NF und rührt 3 Stunden bei RT. Danach verdünnt man mit CH₂Cl₂ und wäscht dreimal mit H₂O. Nach dem Trocknen mit Na₂SO₄ entfernt man das Lösungsmittel am ROT. Der Rückstand (2,1 g) wird ohne weitere Reinigung in Beispiel B6 verwendet. ¹H-NMR (500 MHz, CDCl₃): 3,88 (t, J=7, -CH₂O-), 3,74 (s, -OCH₃).

### Beispiel B6:

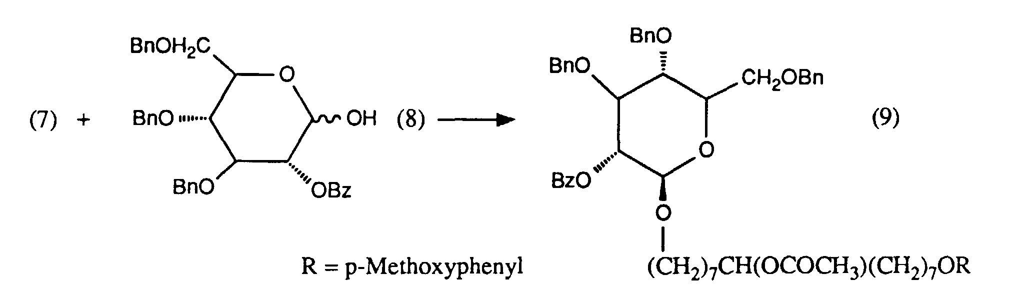

Eine Lösung von 1,5 g (2,7 mMol) (8), 1,23 g (2,7 mMol) (7) und 5 mg (0,14 Mol-%) Katalysator F in 60 ml CH₂Cl₂ werden in einem Rundkolben mit Soxhlet (Hülse mit 4 Angström Molekularsieb) 24 Stunden am Rückfluss erhitzt. Danach entfernt man das Lösungsmittel am ROT und chromatographiert den Rückstand an Kieselgel (Petrolether (40/60)/Essigsäureethylester/8:1). Man erhält 2,34 g (92 %) (9); ¹³C-NMR (500 MHz, CDCl₃): 101,2 (d, C(1)).

### Beispiel B7:

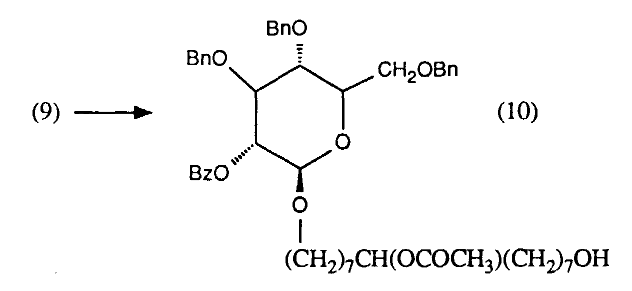

2,08 g (2,2 mMol) (9) und 4,4 g Cerammoniumnitrat werden in 30 ml Acetonitril gelöst, mit 7,5 ml H₂O versetzt und dann bei 0 °C 10 Minuten gerührt. Man verdünnt mit Ether, wäscht je einmal mit H₂O, gesättigter wässriger NaHCO₃ und Sole und trocknet die abgetrennte organische Phase mit Na₂SO₄. Danach destilliert man das Lösungsmittel am ROT ab und chromatographiert den Rückstand an Kieselgel (Petrolether (40/60)/Essigsäureethylester/2:1). Man erhält 1,6 g (10); ¹H-NMR (300 MHz, CDCl₃): 4,51 (d, J=8, anomeres H).

### Beispiel B8:

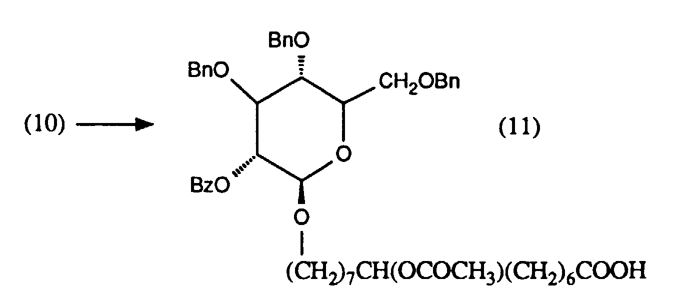

1,54 g (1,84 mMol) (10) werden in 20 ml Aceton gelöst und bei 0 °C mit einer 4N Lösung von CrO₃/H₂SO₄ (1:1) in Aceton versetzt. Danach rührt man 30 Minuten bei 0 °C. Anschliessend verdünnt man mit Ether, filtriert über Watte und wäscht das Filtrat je einmal mit H₂O und Sole. Nach dem Trocknen über Na₂SO₄ destilliert man das Lösungsmittel am ROT ab und chromatographiert den Rückstand an Kieselgel (Petrolether (40/60)/EssigsäureethylesterEssigsäuren/75:22:3). Man erhält 1,18 g (11); ¹H-NMR (300 MHz, CDCl₃): 4,61 (d, J=8, anomeres H).

### Beispiel B9:

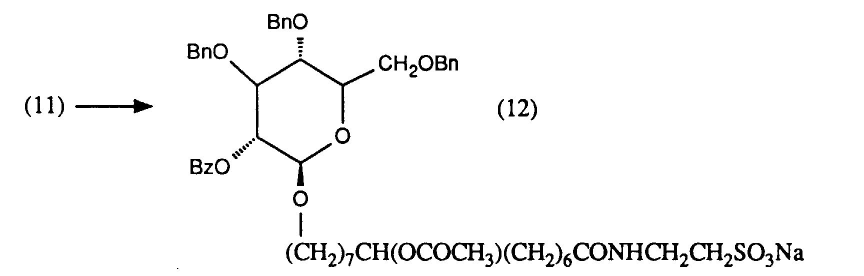

Ein Gemisch von 1,28 g (1,50 mMol) (11), 207 mg N-Hydroxysuccinimid und 402 mg Dicyclohexylcarbodiimid in 10 ml Dimethoxyethan wird bei Raumtemperatur 17 Stunden gerührt. Anschliessend wird das Reaktionsgemisch filtriert, das Filtergut mit 10 ml Dimethoxyethan nachgespült und das Filtrat eingeengt. Der Rückstand wird in 12 ml Methanol gelöst und bei RT mit 188 mg Taurin in 1,58 ml 1N NaOH und 4 ml Methanol versetzt. Man rührt das Gemisch 1,5 Stunden bei RT, engt danach ein und chromatographiert den Rückstand an Kieselgel (Chloroform/Methanol/4:1). Man erhält 1,18 g (12); ¹H-NMR (300 MHz, CDCl₃): 4,46 (d, J=8, anomeres H).

### Beispiel B10:

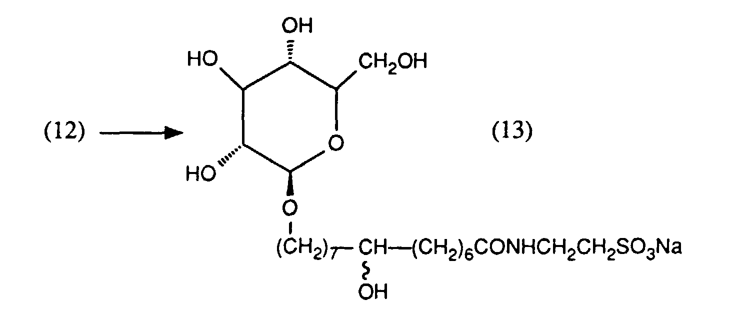

1,26 g (1,28 mMol) (12) in 10 ml Methanol und eine katalytische Menge Na werden bei RT 3 Stunden gerührt. Anschliessend wird das Reaktionsgemisch eingeengt und über Kieselgel filtriert. Das Lösungsmittel wird am ROT entfernt, der Rückstand in Methanol gelöst und mit Pd/C (5 %) unter einer H₂-Atmosphäre 8 Stunden hydriert. Das Gemisch wird dann über Celite filtriert, das Lösungsmittel am ROT entfernt und dann das Rohprodukt an Reversephase-Kieselgel C₁₈ mit Acetonitril/H₂O chromatographiert. Man erhält 628 mg (13); ¹H-NMR (500 MHz, CD₃OD): 3,89 (dt, J₁=9, J₂=7, C-O-CH₂), 3,59 (t,J=7, S-CH₂), 3,53 (dt, J₁=9,5, J₂=7, C-O-CH₂), 3,50 (m, HC-OH), 2,96 (t, J=7, NCH₂), 2,19 (t, J=7,4, -C(O)-CH₂).

## Patentansprüche

1. Verfahren zur Herstellung von Glykosiden durch die Umsetzung von Zuckern mit einer anomeren Hydroxylgruppe und einem Aglykon, dadurch gekennzeichnet, dass man in einem inerten Lösungsmittel einen geschützten Zucker, der eine anomere Hydroxylgruppe enthält, mit einem Aglykon aus der Gruppe a) aliphatische Alkohole, cycloaliphatische Alkohole, aromatische oder aromatisch-aliphatische Alkohole, und b) geschützte Zucker mit einer nicht-anomeren Hydroxylgruppe entweder je alleine oder je zusammen mit einem Orthoester, in Gegenwart katalytischer Mengen eines Metallkomplexsalzes aus (1) einem Metallkation eines Metalls aus der zweiten bis fünften Hauptgruppe, der ersten bis achten Nebengruppe oder der Lanthaniden des Periodensystems der Elemente, (2) einem oder mehreren, gleichen oder verschiedenen, ein- oder mehrzähnigen Liganden entsprechend der Koordinationszahl des Metallkations, (3) gegebenenfalls einem nukleophilen Anion aus der Gruppe Halogenid, Pseudohalogenid, C₁-C₈-Alkoholat oder unsubstituiertes oder mit C₁-C₄-Alkyl substituiertes Phenolat, sekundäres Amido mit 2 bis 12 C-Atomen, Bis-[(Tri-C₁-C₆-Alkyl)silyl]amido oder unsubstituiertes oder mit C₁-C₄-Alkyl substituiertes Cyclopentadienyl, und (4) einem nicht-nukleophilen Anion aus der Gruppe Anion einer Sauerstoffsäure, BF₄, PF₆, AsF₆ oder SbF₆ entsprechend der Wertigkeit des komplexierten Metallkations, umsetzt.

2. Verfahren gemäss Anspruch 1, dadurch gekennzeichnet, dass es sich bei den geschützten Zuckern um geschützte Mono- und Oligosaccharide handelt.

3. Verfahren gemäss Anspruch 2, dadurch gekennzeichnet, dass es sich bei den geschützten Mono- und Oligosacchariden um Aldosen oder Ketosen mit einer anomeren Hydroxylgruppe handelt.

4. Verfahren gemäss Anspruch 3, dadurch gekennzeichnet, dass es sich bei den geschützten Monosacchariden um Aldopyranosen, Aldofuranosen, Ketopyranosen oder Ketofuranosen mit einer anomeren Hydroxylgruppe handelt.

5. Verfahren gemäss Anspruch 1, dadurch gekennzeichnet, dass es sich bei den geschützten Mono- und Oligosaccharid-Derivaten um geschützte Desoxyzucker, Aminozucker, Thiozucker, Zuckersäuren oder Ester von Zuckersäuren handelt, die eine anomere Hydroxylgruppe enthalten.

6. Verfahren gemäss Anspruch 1, dadurch gekennzeichnet, dass sich die Orthoester von einer C₁-C₄-Carbonsäure ableiten.

7. Verfahren gemäss Anspruch 6, dadurch gekennzeichnet, dass sich die Orthoester von Ameisensäure, Essigsäure, Propionsäure oder Buttersäure ableiten.

8. Verfahren gemäss Anspruch 7, dadurch gekennzeichnet, dass sich die Orthoester von Ameisensäure ableiten.

9. Verfahren gemäss Anspruch 6, dadurch gekennzeichnet, dass sich die Orthoester von einer C₁-C₄-Carbonsäure und einem linearen oder verzweigten C₁-C₂₀-Alkanol, C₂-C₂₀-Alkenol mit nicht-vinylischer Alkoholgruppe, einem mono- oder polycycloaliphatischen oder einem mono- oder polycycloheteroaliphatischen C₃-C₂₀-Alkohol mit den Heteroatomen O, S und N, einem aromatischen C₆-C₂₀-Alkohol, oder einem aromatisch-aliphatischen C₇-C₂₀-Alkohol, die unsubstituiert oder mit C₁-C₆-Alkyl, C₁-C₆-Alkoxy oder -Alkylthio, Phenyloxy, C₁-C₄-Alkyl- oder -Alkoxyphenyloxy, Benzyl, Benzyloxy oder C₁-C₄-Alkyl- oder -Alkoxybenzyloxy substituiert sind.

10. Verfahren gemäss Anspruch 9, dadurch gekennzeichnet, dass es sich um Orthoameisensäureester von C₁-C₄-Alkanolen, Allylalkohol, Benzylalkohol und Phenol handelt.

11. Verfahren gemäss Anspruch 1, dadurch gekennzeichnet, dass es sich bei den Aglykonen b) um geschützte Zucker oder Zuckerderivate handelt, deren anomere Hydroxylgruppe geschützt ist und die eine freie Hydroxylgruppe enthalten.

12. Verfahren gemäss Anspruch 11, dadurch gekennzeichnet, dass die Zucker oder Zukkerderivate eine freie Hydroxymethylgruppe enthalten.

13. Verfahren gemäss Anspruch 1, dadurch gekennzeichnet, dass die Metallkomplexsalze in einer Menge von 0,01 bis 20 Mol.-% verwendet werden, bezogen auf die Menge des geschützten Zuckers oder Zuckerderivates.

14. Verfahren gemäss Anspruch 13, dadurch gekennzeichnet, dass die Metallkomplexsalze in einer Menge von 0,01 bis 10 Mol.-% verwendet werden, bezogen auf die Menge des geschützten Zuckers oder Zuckerderivates.

15. Verfahren gemäss Anspruch 1, dadurch gekennzeichnet, dass sich die Metallkationen von folgenden Metallen aus den genannten Gruppen des Periodensystems der Elemente ableiten: Mg, Ca, Sr, Ba; B, Al, Ga, In; Sn, Pb; Sb, Bi; Cu, Ag, Au; Zn, Cd, Hg; Sc, Y, La; Ti, Zr, Hf; V, Nb, Ta; Cr, Mo, W; Mn; Fe, Co, Ni, Ru, Rh, Pd, Os, Ir, Pt; und den Lanthanidenmetallen Ce, Pr, Nd, Pm, Sm, Eu, Gd, Tb, Dy, Ho, Er, Tm, Yb und Lu.

16. Verfahren gemäss Anspruch 15, dadurch gekennzeichnet, dass sich die Metallkationen von folgenden Metallen ableiten: Mg, Ca, B, In, Sn, Pb, Cu, Ag, Au, Zn, Ti, Zr, V, Cr, Mo, W, Mn, Fe, Co, Ni, Ru, Rh, Pd, Ir, Pt, Ce, Nd und Yb.

17. Verfahren gemäss Anspruch 1, dadurch gekennzeichnet, dass der Ligand achiral oder chiral ist.

18. Verfahren gemäss Anspruch 1, dadurch gekennzeichnet, dass der Ligand ein bis dreizähnig ist.

19. Verfahren gemäss Anspruch 1, dadurch gekennzeichnet, dass die mehrzähnigen Liganden mit den komplexbildenden Gruppen und dem Metallkation einen fünf- bis siebenglidrigen Ring bilden.

20. Verfahren gemäss Anspruch 1, dadurch gekennzeichnet, dass die komplexbildenden Gruppen der mehrzähnigen Liganden in den 1,2-, 1,3- oder 1,4-Stellungen einer Kohlenstoffkette mit 2 bis 4 C-Atomen gebunden sind.

21. Verfahren gemäss Anspruch 1, dadurch gekennzeichnet, dass es sich bei den Liganden um schwach koordinierende organische Liganden mit 1 bis 20 bevorzugt 1 bis 12 C-Atomen, die zur Koordination befähigte Heteroatome oder Heterogruppen enthalten, oder um H₂O handelt.

22. Verfahren gemäss Anspruch 21, dadurch gekennzeichnet, dass sich es bei den Heteroatomen oder Heterogruppen um -OH, -CN, -CHO, -CO-, -O-, -C(O)OR₀ und P(O-)₃ handelt, worin R₀ der Rest eines C₁-C₁₂-Alkohols ist.

23. Verfahren gemäss Anspruch 21, dadurch gekennzeichnet, dass es sich bei den schwach koordinierenden organischen Liganden um Alkohole mit 1 bis 12 C-Atomen, Nitrile, Isonitrile, Aldehyde, Ketone, C₁-C₄-Alkylester von aliphatischen Carbonsäuren, Ether oder Phosphite handelt.

24. Verfahren gemäss Anspruch 23, dadurch gekennzeichnet, dass es sich bei den schwach koordinierenden organischen Liganden um Acetonitril, Benzonitril, Methanol, Ethanol, Phenol, Aceton, Pivaldehyd, Essigsäureethylester, Diethylether, Tetrahydrofuran und Dioxan sowie um Wasser handelt.

25. Verfahren gemäss Anspruch 1, dadurch gekennzeichnet, dass die Metallkomplexsalze nur schwach koordinierende Liganden, nur stark kordinierende Liganden oder beide Ligandenarten enthalten.

26. Verfahren gemäss Anspruch 1, dadurch gekennzeichnet, dass die stark koordinierenden Liganden organische Verbindungen sind, die 2 bis 30 C-Atome enthalten, und als komplexbildende Gruppen ein oder mehrere, gleiche oder verschiedene, gegebenenfalls substituierte Amino-, Phosphino-, Phosphonit-, Arsino- oder Stibinogruppen enthalten.

27. Verfahren gemäss Anspruch 26, dadurch gekennzeichnet, dass die Amino-, Phosphino-, Phosphonit-, Arsino- oder Stibinogruppen mit C₁-C₁₂-Alkyl, C₅- oder C₆-Cycloalkyl, Phenyl oder Benzyl substituiert sind, die ihrerseits unsubstituiert oder mit C₁-C₄-Alkyl oder C₁-C₄-Alkoxy substituiert sind.

28. Verfahren gemäss Anspruch 1, dadurch gekennzeichnet, dass es sich bei den einzähnigen stark koordinierenden Liganden um tertiäre Amine, Phosphine, Arsine oder Stibine handelt.

29. Verfahren gemäss Anspruch 28, dadurch gekennzeichnet, dass es sich bei den einzähnigen stark koordinierenden Liganden um solche der Formel XR₁R₂R₃ handelt, worin X für N, P, As oder Sb steht und R₁, R₂ und R₃ unabhängig voneinander C₁-C₁₂-Alkyl, C₅-oder C₆-Cycloalkyl, Phenyl oder Benzyl bedeuten, oder R₁ und R₂ zusammen Tetramethylen, Pentamethylen oder 3-Oxa-1,5-pentylen darstellen und R₃ die zuvor angegebene Bedeutung hat, die unsubstituiert oder mit C₁-C₄-Alkyl oder C₁-C₄-Alkoxy substituiert sind.

30. Verfahren gemäss Anspruch 28, dadurch gekennzeichnet, dass es sich bei den einzähnigen stark koordinierenden Liganden um tertiäre Phosphine handelt.

31. Verfahren gemäss Anspruch 1, dadurch gekennzeichnet, dass die komplexbildenden Gruppen in den stark koordinierenden mehrzähnigen Liganden den Formeln -NR₁R₂, -PR₁R₂, -OPR₁R₂, -AsR₁R₂ oder -SbR₁R₂ entsprechen, worin R₁ und R₂ unabhängig voneinander C₁-C₁₂-ALkyl, C₅- oder C₆-Cycloalkyl, Phenyl oder Benzyl sind, die unsubstituiert oder mit C₁-C₄-Alkyl oder C₁-C₄-Alkoxy substituiert sind.

32. Verfahren gemäss Anspruch 31, dadurch gekennzeichnet, dass die komplexbildende Gruppe der Formel -PR₁R₂ entspricht.

33. Verfahren gemäss Anspruch 1, dadurch gekennzeichnet, dass die dreizähnigen stark komplexierenden Liganden Phosphine der Formel R₄C(CH₂PR₁R₂)₃ sind, worin R₁ und R₂ die im Anspruch 31 angegebenen Bedeutungen haben, und R₄ für H, C₁-C₄-Alkyl, Phenyl oder Benzyl steht, oder N,N',N"-pentaalkylierte Diethylentriamine sind, wobei die Alkylgruppen 1 bis 4 C-Atome enthalten.

34. Verfahren gemäss Anspruch 33, dadurch gekennzeichnet, dass es sich bei den dreizähnigen stark komplexierenden Liganden um CH₃-C[CH₂P(C₆H₅)₂]₃, Bis-[(2-diphenylphosphino)ethyl]phenylphosphin oder N,N',N"-Pentamethyl-diethylentriamin handelt.

35. Verfahren gemäss Anspruch 1, dadurch gekennzeichnet, dass es sich bei den vierzähnigen stark komplexierenden Liganden um C[CH₂P(C₆H₅)]₄, Tri[(2-diphenylphosphino)ethyl]phosphin, Tri[(2-dimethylamino)ethyl]amin oder N,N',N",N‴-Hexamethyl-triethylentetraamin handelt.

36. Verfahren gemäss Anspruch 1, dadurch gekennzeichnet, dass es sich bei den zweizähnigen stark komplexierenden Liganden um aliphatische, cycloaliphatische, cycloheteroaliphatische, aromatische oder heteroaromatische Verbindungen mit 2 bis 30 handelt (ohne die C-Atome in den komplexbildenden Gruppen), die gleiche oder verschiedene der komlexbildenden Gruppen enthalten, wobei die komplexbildenden Gruppen in den aliphatischen Verbindungen in 1,2-, 1,3- oder 1,4-Stellung und in den cyclischen Verbindungen in 1,2- oder 1,3-Stellung gebunden sind, und wobei die Heteroatome aus der Gruppe O, S und N ausgewählt sind und wobei 1 oder 2 Heteroatome enthalten sind, und wobei die komplexbildenden Gruppen direkt oder über eine -CR₅R₆-Gruppe an das Grundgerüst gebunden sind, und R₅ und R₆ unabhängig voneinander H, C₁-C₄-Alkyl, Phenyl oder Benzyl bedeuten.

37. Verfahren gemäss Anspruch 36, dadurch gekennzeichnet, dass es sich bei den komplexbildende Gruppen um solche der Formeln -NR₁R₂, -PR₁R₂, -OPR₁R₂, -AsR₁R₂ oder -SbR₁R₂ handelt, worin R₁ und R₂ unabhängig voneinander C₁-C₁₂-Alkyl, C₅- oder C₆-Cycloalkyl, Phenyl oder Benzyl sind, die unsubstituiert oder mit C₁-C₄-Alkyl oder C₁-C₄-Alkoxy substituiert sind.

38. Verfahren gemäss Anspruch 36, dadurch gekennzeichnet, dass die zweizähnigen Liganden der Formel I entsprechen,
R₁R₂X-Y-X'R₁R₂ (I),
worin X und X' unabhängig voneinander für N, P, As, Sb oder -OP stehen, R₁ und R₂ unabhängig voneinander C₁-C₁₂-Alkyl, C₅- oder C₆-Cycloalkyl, Phenyl oder Benzyl bedeuten, die unsubstituiert oder mit C₁-C₄-Alkyl oder C₁-C₄-Alkoxy substituiert sind, Y einen linearen oder verzweigten Alkylenrest oder Alkenylenrest mit 2 bis 20 C-Atomen darstellt, der unsubstituiert oder mit C₁-C₆-Alkoxy, C₅- oder C₆-Cycloalkyl, C₅- oder C₆-Cycloalkoxy, Phenyl, Phenyloxy, Benzyl oder Benzyloxy substituiert ist, und die cyclischen Substituenten ihrerseits unsubstituiert oder mit C₁-C₆-Alkyl oder C₁-C₆-Alkoxy substituiert sind, und die Gruppen R₁R₂X- beziehungsweise R₁R₂X'- im Alkylenrest oder Alkenylenrest in α,β-, α,γ- oder α,δ-Stellung gebunden sind.

39. Verfahren gemäss Anspruch 36, dadurch gekennzeichnet, dass die zweizähnigen Liganden der Formel II entsprechen, 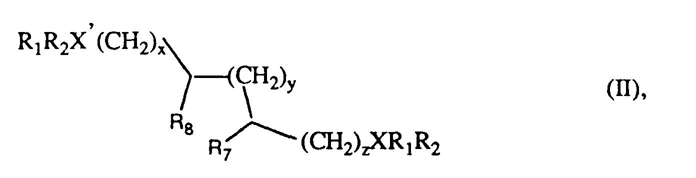 worin X und X' unabhängig voneinander für N, P, As, Sb oder -OP stehen, R₁ und R₂ unabhängig voneinander C₁-C₁₂-Alkyl, C₅- oder C₆-Cycloalkyl, Phenyl oder Benzyl bedeuten, die unsubstituiert oder mit C₁-C₄-Alkyl oder C₁-C₄-Alkoxy substituiert sind, x, y und z unabhängig voneinander für 0 oder 1 stehen, wobei die Summe von x+y+z 0, 1 oder 2 beträgt, R₇ und R₈ zusammen mit dem Rest, an den sie gebunden sind, einen 5- oder 6- gliedrigen cycloaliphatischen Rest oder einen 5- oder 6-gliedrigen cycloheteroaliphatischen Rest mit ein oder zwei gleichen oder verschiedenen Heteroatomen aus der Gruppe O, S und NR₉ bilden, die unsubstituiert oder mit C₁-C₆-Alkyl oder C₁-C₆-Alkoxy oder Halogen substituiert sind, R₉ H, C₁-C₆-Alkyl, C₁-C₈-Acyl, oder unsubstituiertes oder mit C₁-C₆-Alkyl oder C₁-C₆-Alkoxy substituiertes Phenyl oder Benzyl darstellt, und wenn y für 0 steht, R₇ und R₈ zusammen mit dem Rest, an den sie gebunden sind, einen 6-gliedrigen aromatischen oder heteroaromatischen Rest oder einen 5-gliedrigen heteroaromatischen Rest bilden, die unsubstiuiert oder mit C₁-C₆-Alkyl oder C₁-C₆-Alkoxy oder Halogen substituiert sind.

40. Verfahren gemäss Anspruch 1, dadurch gekennzeichnet, dass es sich bei den zweizähnigen stark komplexierenden Liganden um in den o,o'-Stellungen mit gleichen oder verschiedenen Gruppen -XR₁R₂ substituiertes Biphenylen oder Naphthylen handelt, worin die X unabhängig voneinander für N, P, As, Sb oder -OP stehen, R₁ und R₂ unabhängig voneinander C₁-C₁₂-C₆-Alkyl, C₅- oder C₆-Cycloalkyl, Phenyl oder Benzyl sind, die unsubstituiert oder mit C₁-C₄-Alkyl oder C₁-C₄-Alkoxy substituiert sind.

41. Verfahren gemäss Anspruch 40, dadurch gekennzeichnet, dass es sich um 2,2'-Diphenylphosphino- oder -Dicyclohexylphosphinobiphenyl oder -binaphthyl handelt.

42. Verfahren gemäss Anspruch 36, dadurch gekennzeichnet, dass die zweizähnigen Liganden der Formel II entsprechen, 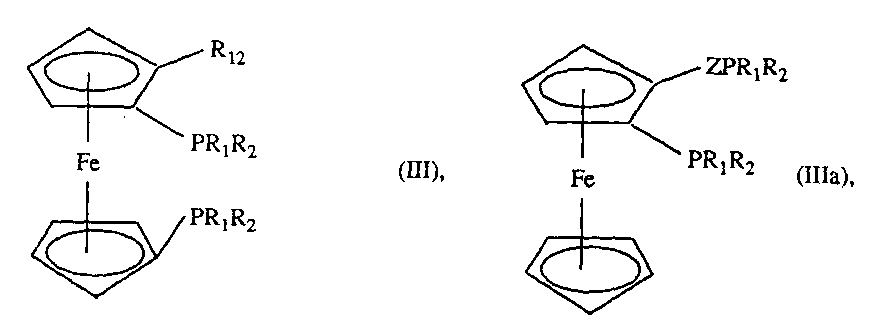 worin R₁ und R₂ unabhängig voneinander C₁-C₁₂-Alkyl, C₅- oder C₆-Cycloalkyl, Phenyl oder Benzyl darstellen, die unsubstituiert oder mit C₁-C₄-Alkyl oder C₁-C₄-Alkoxy substituiert sind, R₁₂ für H, C₁-C₆-Alkyl, C₁-C₆-Alkenyl, Tri(C₁-C₆-Alkyl)silyl, Phenyl, Benzyl, α-Hydroxy- oder α- [Di(C₁-C₆-Alkyl)amino]-C₁-C₆-alkyl steht, und Z Methylen oder C₁-C₈-Alkyliden bedeutet.

43. Verfahren gemäss Anspruch 36, dadurch gekennzeichnet, dass es sich bei den zweizähnigen Liganden um unsubstituiertes oder mit C₁-C₄-Alkyl, C₁-C₄-Alkoxy, F oder Cl substituiertes α,α'-Bipyridyl handelt.

44. Verfahren gemäss Anspruch 36, dadurch gekennzeichnet, dass es sich bei den zweizähnigen Liganden um Bisoxazolidine der Formel IV handelt, 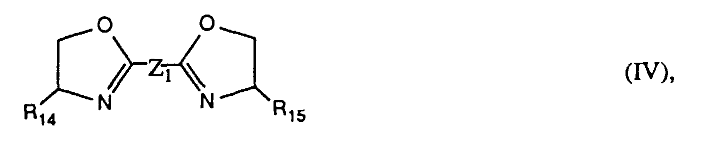 worin R₁₄ und R₁₅ gleich oder verschieden sind und H, lineares oder verzweigtes C₁-C₆-Alkyl, Phenyl, (C₁-C₆-Alkyl)phenyl, Benzyl oder (C₁-C₆-Alkyl)benzyl darstellen, und Z₁ für eine direkte Bindung, Methylen, C₂-C₈-Alkyliden, Ethylen, 1,2-Propylen oder 1,2-Phenylen steht.

45. Verfahren gemäss Anspruch 36, dadurch gekennzeichnet, dass es sich bei den zweizähnigen Liganden um Bisoxazolidine der Formel V handelt, 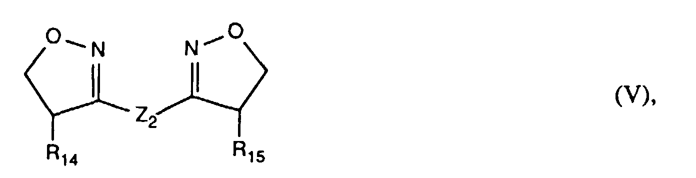 worin R₁₄ und R₁₅ gleich oder verschieden sind und H, lineares oder verzweigtes C₁-C₆-Alkyl, Phenyl, (C₁-C₆-Alkyl)phenyl, Benzyl oder (C₁-C₆-Alkyl)benzyl darstellen, und Z₂ für eine direkte Bindung, Methylen, 1,2- oder 1,3-Phenylen, pydin-2,6-diyl, Ethylen, 1,2-oder 1,3-Propylen oder 1,2-, 1,3-, 1,4- oder 2,3-Butylen oder C₂-C₈-Alkyliden steht.

46. Verfahren gemäss Anspruch 1, dadurch gekennzeichnet, dass die Metallkomplexsalze zwei verschiedene stark koordinierende zweizähnige Liganden, einen zweizähnigen stark koordinierenden Liganden und einen einzähnigen stark koordinierenden Liganden, einen zweizähnigen stark koordinierenden Liganden und zwei einzähnige schwach oder stark koordinierende Liganden, zwei verschiedene dreizähnige stark koordinierende Liganden oder einen dreizähnigen stark koordinierenden Liganden und drei einzähnige schwach oder stark koordinierende Liganden enthalten.

47. Verfahren gemäss Anspruch 1, dadurch gekennzeichnet, dass die Metallkomplexsalze als nukleophile koordinierende Anionen ein Halogenid enthalten.

48. Verfahren gemäss Anspruch 1, dadurch gekennzeichnet, dass die Metallkomplexsalze als Anionen von Sauerstoffsäuren Sulfat, Phosphat, Perchlorat, Perbromat, Periodat, Antimonat, Arsenat, Nitrat, Carbonat, das Anion einer C₁-C₈-Carbonsäure, Sulfonate und Phosphonate enthalten.

49. Verfahren gemäss Anspruch 1, dadurch gekennzeichnet, dass die Metallkomplexsalze als Anionen Tetrafluorborat, Hexafluorphosphat, Perchlorat oder Triflat enthalten.

50. Verfahren gemäss Anspruch 1, dadurch gekennzeichnet, dass die Metallkomplexsalze der Formel VI entsprechen,
[(Q)ₘ(M⁺ⁿ)(S)ₒ(L⁻¹)_{q}]^{+(n-q)}(A^{-r})_{(n-q)/r} (VI),
worin Q gleiche oder verschiedene stark koordinierende Liganden bedeutet, m für 0 steht oder Q ein einzähniger Ligand und m eine Zahl von 1 bis 8, Q ein zweizähniger Ligand und m eine Zahl von 1 bis 4, Q ein dreizähniger Ligand und m die Zahlen 1 oder 2 oder Q ein vierzähniger Ligand und m die Zahlen 1 oder 2 sind, wobei die zwei-, drei- und vierzähnigen Liganden ein oder mehrere gleiche oder verschiedene Phosphin-, Phosphonit-, Arsin-, oder Stibingruppen oder primäre, sekundäre und/oder tertiäre Amingruppen und/oder Imingruppen als komplexbildende Gruppen enthalten, und wobei diese Liganden mit dem Metallkation M⁺ⁿ einen 5- bis 7-gliedrigen, bevorzugt einen 5- oder 6-gliedrigen Ring bilden; M ein Metall aus der zweiten bis fünften Hauptgruppe, der ersten bis achten Nebengruppe oder der Lanthaniden im Periodensystem der Elemente darstellt und n eine Zahl von 1 bis 4 ist; S ein schwach koordinierender Ligand aus der Gruppe Nitrile, aliphatische oder aromatische Alkohole, Ketone, Aldehyde, Carbonsäureester, Ether, Tris(trifluormethyl)phosphit, und Wasser bedeutet und o für 0 oder eine Zahl von 1 bis 6 steht; L Halogenid, Pseudohalogenid, C₁-C₈-Alkoholat oder unsubstituiertes oder mit C₁-C₄-Alkyl substituiertes Phenolat, sekundäres Amid mit 2 bis 12 C-Atomen, Bis-[(Tri-C₁-C₆-Alkyl)silyl] amid oder unsubstituiertes oder mit C₁-C₄-Alkyl substituiertes Cyclopentadienyl bedeutet und q für 0 oder eine Zahl von 1 bis 4 steht; A ein Anion einer Sauerstoffsäure, BF₄, PF₆, AsF₆ oder SbF₆ darstellt und r für eine Zahl von 1 bis 3 steht, wobei die Summe von (m x Zähnigkeit des Liganden Q) + o + q eine ganze Zahl von 2 bis 8 ist und die Summe von (m x Zähnigkeit des Liganden Q) + o mindestens 2 ist.

51. Verfahren gemäss Anspruch 1, dadurch gekennzeichnet, dass man als Lösungsmittel halogenierte aliphatische Kohlenwasserstoffe, aromatische Kohlenwasserstoffe, Ether und Nitrile verwendet.

52. Verfahren gemäss Anspruch 1, dadurch gekennzeichnet, dass der geschützte Zucker und das Aglykon in äquimolaren Mengen verwendet werden.

53. Verfahren gemäss Anspruch 1, dadurch gekennzeichnet, dass der Orthoester in einer Menge von 1,2 bis 10 Äquivalenten pro Äquivalent geschütztem Zucker eingesetzt wird.

54. Verfahren gemäss Anspruch 1, dadurch gekennzeichnet, dass die Menge 1,2 bis 5 Äquivalente beträgt.

55. Verfahren gemäss Anspruch 1, dadurch gekennzeichnet, dass die Reaktionstemperatur -20 bis 250 °C beträgt.

56. Verfahren gemäss Anspruch 1, dadurch gekennzeichnet, dass die Reaktionstemperatur 10 bis 200 °C beträgt.

57. Verfahren gemäss Anspruch 1, dadurch gekennzeichnet, dass man einen Orthoester mitverwendet und die Reaktion bei Raumtemperatur durchführt.

58. Verfahren gemäss Anspruch 1, dadurch gekennzeichnet, dass man das entstehende Reaktionswasser chemisch bindet oder durch Destillation oder Verwendung von Wasser absorbierenden Mitteln entfernt.

59. Verfahren gemäss Anspruch 1, dadurch gekennzeichnet, dass man den geschützten Zucker und den Katalysator in einem Lösungsmittel bei Raumtemperatur vorlegt, dann das Aglykon und einen Orthoester zugibt, und dann das Gemisch bei Raumtemperatur bis Rückflusstemperatur der Lösungsmittel rührt.

60. Verfahren gemäss Anspruch 59, dadurch gekennzeichnet, dass man Orthoester von Alkoholen verwendet, die gleichzeitig als Aglykon eingesetzt werden.

61. Verfahren gemäss Anspruch 1, dadurch gekennzeichnet, dass man den geschützten Zucker und das Aglykon in einem Lösungsmittel vorlegt, dann den Katalysator zugibt, das Wasser absorbierende Mittel in einem Soxhlet in einer Hülse vorlegt, und dann das Reaktionsgemisch am Rückfluss erhitzt.

62. Verfahren gemäss Anspruch 1, dadurch gekennzeichnet, dass man den geschützten Zucker und das Aglykon in einem Lösungsmittel vorlegt, dann den Katalysator zugibt, wobei man eine Apparatur mit Wasserabscheider verwendet, dann das Reaktionsgemisch am Rückfluss erhitzt und das Reaktionswasser azeotrop abdestilliert, bis die Reaktion beendet ist.

## Claims

1. A process for the preparation of glycosides by reacting sugars carrying an anomeric hydroxyl group with an aglycon, which process comprises reacting, in an inert solvent, a protected sugar which carries an anomeric hydroxyl group with an aglycon selected from the group consisting of a) aliphatic alcohols, cycloaliphatic alcohols, aromatic or aromatic-aliphatic alcohols, and b) protected sugars carrying a non-anomeric hydroxyl group, either in each case alone or in each case together with an orthoester, in the presence of catalytic amounts of a metal complex salt of (1) a metal cation of a metal of the second to fifth main group, of the first to eighth subgroup, or of the lanthanides, of the Periodic Table of the Elements, (2) one or more identical or different monodentate or polydentate ligands in accordance with the co-ordination number of the metal cation, (3) optionally a nucleophilic anion selected from the group consisting of halide, pseudohalide, C₁-C₈alcoholate or unsubstituted or C₁-C₄alkyl-substituted phenolate, secondary amido containing 2 to 12 carbon atoms, bis[(tri-C₁-C₆alkyl) silyl]amido or unsubstituted or C₁-C₄alkyl-substituted cyclopentadienyl, and (4) a non-nucleophilic anion selected from the group consisting of anion of an oxyacid, BF₄, PF₆, AsF₆ or SbF₆ in accordance with the valency of the complexed metal cation.

2. A process according to claim 1, wherein the protected sugars are protected mono- and oligosaccharides.

3. A process according to claim 2, wherein the protected mono- and oligosaccharides are aldoses or ketoses which carry an anomeric hydroxyl group.

4. A process according to claim 3, wherein the protected monosaccharides are aldopyranoses, aldofuranoses, ketopyranoses or ketofuranoses which carry an anomeric hydroxyl group.

5. A process according to claim 1, wherein the protected mono- and oligosaccharide derivatives are protected deoxy sugars, amino sugars, thiosugars, sugar acids or esters of sugar acids which carry an anomeric hydroxyl group.

6. A process according to claim 1, wherein the orthoesters are derived from a C₁-C₄carboxylic acid.

7. A process according to claim 6, wherein the orthoesters are derived from formic acid, acetic acid, propionic acid or butyric acid.

8. A process according to claim 7, wherein the orthoesters are derived from formic acid.

9. A process according to claim 6, wherein the orthoesters are derived from a C₁-C₄carboxylic acid and a linear or branched C₁-C₂₀alkanol, a C₂-C₂₀alkenol carrying a non-vinylic alcohol group, a mono- or polycycloaliphatic or a mono- or polycycloheteroaliphatic C₃-C₂₀alcohol containing the heteroatoms O, S and N, an aromatic C₆-C₂₀alcohol, or an aromatic-aliphatic C₇-C₂₀alcohol, which are unsubstituted or substituted by C₁-C₆alkyl, C₁-C₆alkoxy or C₁-C₆alkylthio, phenoxy, C₁-C₄alkylphenoxy or C₁-C₄alkoxyphenoxy, benzyl, benzyloxy or C₁-C₄alkylbenzyloxy or C₁-C₄alkoxybenzyloxy.

10. A process according to claim 9, wherein the ortho-esters are orthoformates of C₁-C₄alkanols, allyl alcohol, benzyl alcohol and phenol.

11. A process according to claim 1, wherein the aglycons b) are protected sugars or sugar derivatives whose anomeric hydroxyl group is protected, which contain a free hydroxyl group.

12. A process according to claim 11, wherein the sugars or sugar derivatives contain a free hydroxymethyl group.

13. A process according to claim 1, wherein the metal complex salts are used in an amount of 0.01 to 20 mol %, based on the amount of the protected sugar or sugar derivative.

14. A process according to claim 13, wherein the metal complex salts are used in an amount of 0.01 to 10 mol %, based on the amount of the protected sugar or sugar derivative.

15. A process according to claim 1, wherein the metal cations are derived from the following metals of the cited groups of the Periodic Table of the Elements: Mg, Ca, Sr, Ba; B, Al, Ga, In; Sn, Pb; Sb, Bi; Cu, Ag, Au; Zn, Cd, Hg; Sc, Y, La; Ti, Zr, Hf; V, Nb, Ta; Cr, Mo, W; Mn; Fe, Co, Ni, Ru, Rh, Pd, Os, Ir, Pt; and the lanthanide metals Ce, Pr, Nd, Pm, Sm, Eu, Gd, Tb, Dy, Ho, Er, Tm, Yb and Lu.

16. A process according to claim 15, wherein the metal cations are derived from the following metals: Mg, Ca, B, In, Sn, Pb, Cu, Ag, Au, Zn, Ti, Zr, V, Cr, Mo, W, Mn, Fe, Co, Ni, Ru, Rh, Pd, Ir, Pt, Ce, Nd and Yb.

17. A process according to claim 1, wherein the ligand is a chiral or chiral.

18. A process according to claim 1, wherein the ligand is monodentate to tridentate.

19. A process according to claim 1, wherein the polydentate ligands form with the complex-forming groups and the metal cation a five- to seven-membered ring.

20. A process according to claim 1, wherein the complex-forming groups of the polydentate ligands are in the 1,2-, 1,3- or 1,4-positions of a carbon chain containing 2 to 4 carbon atoms.

21. A process according to claim 1, wherein the ligands are weakly co-ordinating organic ligands which contain 1 to 20, preferably 1 to 12, carbon atoms, and which contain hetero-groups or heteroatoms capable of co-ordination, or are H₂O.

22. A process according to claim 21, wherein the heteroatoms or hetero-groups are -OH, -CN, -CHO, -CO-, -O-, -C(O)OR₀ and P(O-)₃, where R₀ is the radical of a C₁-C₁₂alcohol.

23. A process according to claim 21, wherein the weakly co-ordinating organic ligands are alcohols of 1 to 12 carbon atoms, nitriles, isonitriles, aldehydes, ketones, C₁-C₄alkyl esters of aliphatic carboxylic acids, ethers or phosphites.

24. A process according to claim 23, wherein the weakly co-ordinating organic ligands are acetonitrile, benzonitrile, methanol, ethanol, phenol, acetone, pivalaldehyde, ethyl acetate, diethyl ether, tetrahydrofuran, dioxane and water.

25. A process according to claim 1, wherein the metal complex salts contain only weakly co-ordinating ligands, only strongly co-ordinating ligands or both types of ligand.

26. A process according to claim 1, wherein the strongly co-ordinating ligands are organic compounds which contain 2 to 30 carbon atoms, and, as complex-forming groups, contain one or more identical or different, unsubstituted or substituted amino, phosphino, phosphonite, arsino or stibino groups.

27. A process according to claim 26, wherein the amino, phosphino, phosphonite, arsino or stibino groups are substituted by C₁-C₁₂alkyl, C₅cycloalkyl or C₆cycloalkyl, phenyl or benzyl which are in turn unsubstituted or substituted by C₁-C₄alkyl or C₁-C₄alkoxy.

28. A process according to claim 1, wherein the monodentate strongly co-ordinating ligands are tertiary amines, phosphines, arsines or stibines.

29. A process according to claim 28, wherein the monodentate strongly co-ordinating ligands are those of formula XR₁R₂R₃, where X is N, P, As or Sb and R₁, R₂ and R₃ are each independently of one another C₁-C₁₂alkyl, C₅cycloalkyl or C₆cycloalkyl, phenyl or benzyl, or R₁ and R₂, when taken together, are tetramethylene, pentamethylene or 3-oxa-1,5-pentylene, and R₃ is as previously defined, which are unsubstituted or substituted by C₁-C₄alkyl or C₁-C₄alkoxy.

30. A process according to claim 28, wherein the monodentate strongly co-ordinating ligands are tertiary phosphines.

31. A process according to claim 1, wherein the complex-forming groups in the strongly co-ordinating polydentate ligands have the formula -NR₁R₂, -PR₁R₂,-OPR₁R₂, -AsR₁R₂ or -SbR₁R₂ where R₁ and R₂ are each independently of the other C₁-C₁₂alkyl, C₅cycloalkyl or C₆cycloalkyl, phenyl or benzyl which are unsubstituted or substituted by C₁-C₄alkyl or C₁-C₄alkoxy.

32. A process according to claim 31, wherein the complex-forming group has the formula -PR₁R₂.

33. A process according to claim 1, wherein the tridentate strongly complexing ligands are phosphines of formula R₄C(CH₂PR₁R₂)₃, wherein R₁ and R₂ are as defined in claim 31, and R₄ is H, C₁-C₄alkyl, phenyl or benzyl or N,N'N"-pentaalkylated diethylenetriamines in which the alkyl groups contain 1 to 4 carbon atoms.

34. A process according to claim 33, wherein the tridentate strongly complexing ligands are CH₃-C [CH₂P(C₆H₅)₂]₃, bis[2-diphenylphosphino)ethyl]phenylphosphine or N,N',N"-pentamethyldiethylenetriamine.

35. A process according to claim 1, wherein the tetradentate strongly complexing ligands are C [CH₂P(C₆H₅)₄, tri [(2-diphenylphosphino)ethyl]phosphine, tri [2-dimethylamino)ethyl]amine or N,N',N",N"'-hexamethyltriethylenetetraamine.

36. A process according to claim 1, wherein the bidentate strongly complexing ligands are aliphatic, cycloaliphatic, cycloheteroaliphatic, aromatic or heteroaromatic compounds containing 2 to 30 carbon atoms (without the carbon atoms in the complex-forming groups) and containing identical or different complex-forming groups, the complex-forming groups in the aliphatic compounds being in 1,2-, 1,3- or 1,4-position and, in the cyclic compounds, in 1,2- or 1,3-position, and the heteroatoms being selected from the group consisting of O, S and N, and the number of heteroatoms being preferably 1 or 2, and the complex-forming groups being attached directly or through a -CR₅R₆ group to the molecule framework, and R₅ and R₆ being each independently of the other H, C₁-C₄alkyl, phenyl or benzyl.

37. A process according to claim 36, wherein the complex-forming groups are those of formula -NR₁R₂, -PR₁R₂, -OPR₁R₂, -AsR₁R₂ or -SbR₁R₂, where R₁ and R₂ are each independently of the other C₁-C₁₂alkyl, C₅cycloalkyl or C₆cycloalkyl, phenyl or benzyl which are unsubstituted or substituted by C₁-C₄alkyl or C₁-C₄alkoxy.

38. A process according to claim 36, wherein the bidentate ligands have the formula I
R₁R₂X-Y-X'R₁R₂ (I),
wherein X and X' are each independently of the other N, P, As, Sb or -OP, R₁ and R₂ are each independently of the other C₁-C₁₂alkyl, C₅cycloalkyl or C₆cycloalkyl, phenyl or benzyl which are unsubstituted or substituted by C₁-C₄alkyl or C₁-C₄alkoxy, Y is a linear or branched alkylene or alkenylene radical of 2 to 20 carbon atoms, which is unsubstituted or substituted by C₁-C₆alkoxy, C₅cycloalkyl or C₆cycloalkyl, C₅cycloalkoxy or C₆cycloalkoxy, phenyl, phenoxy, benzyl or benzyloxy, and the cyclic substituents are in turn unsubstituted or substituted by C₁-C₆alkyl or C₁-C₆alkoxy, and the groups R₁R₂X- and R₁R₂X'- are in α,β-, α,γ- or α,δ-position in the alkylene or alkenylene radical.

39. A process according to claim 36, wherein the bidentate ligands have the formula II 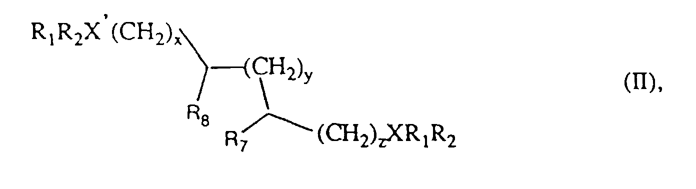 wherein X and X' are each independently of the other N, P, As, Sb or -OP, R₁ and R₂ are each independently of the other C₁-C₁₂alkyl, C₅cycloalkyl or C₆-cycloalkyl, phenyl or benzyl which are unsubstituted or substituted by C₁-C₄alkyl or C₁-C₄alkoxy, x, y and z are each independently of one another 0 or 1, the sum of x+y+z being 0, 1 or 2, R₇ and R₈, together with the radical to which they are attached, form a 5- or 6-membered cycloaliphatic radical or a 5- or 6-membered cycloheteroaliphatic radical containing one or two identical or different heteroatoms selected from the group consisting of O, S and NR₉, which radicals are unsubstituted or substituted by C₁-C₆alkyl or C₁-C₆alkoxy or halogen, R₉ is H, C₁-C₆alkyl, C₁-C₈acyl, phenyl or benzyl or phenyl or benzyl which are substituted by C₁-C₆alkyl or C₁-C₆alkoxy, and, if y is 0, R₇ and R₈, together with the radical to which they are attached, form a 6-membered aromatic or heteroaromatic radical or a 5-membered heteroaromatic radical, which radicals are unsubstituted or substituted by C₁-C₆alkyl or C₁-C₆alkoxy or halogen.

40. A process according to claim 1, wherein the bidentate strongly complexing ligands are biphenylene or naphthylene which are substituted in the o,o'-positions by identical or different groups -XR₁R₂, where the substituents X are each independently of one another N, P, As, Sb or -OP, R₁ and R₂ are each independently of the other C₁-C₁₂alkyl, C₅cycloalkyl or C₆cycloalkyl, phenyl or benzyl which are unsubstituted or substituted by C₁-C₄alkyl or C₁-C₄alkoxy.

41. A process according to claim 40, wherein the ligands are 2,2'-diphenylphosphinobiphenyl or -binaphthyl or 2,2'-dicyclohexylphosphinobiphenyl or -binaphthyl.

42. A process according to claim 36, wherein the bidentate ligands have the formula III or IIIa 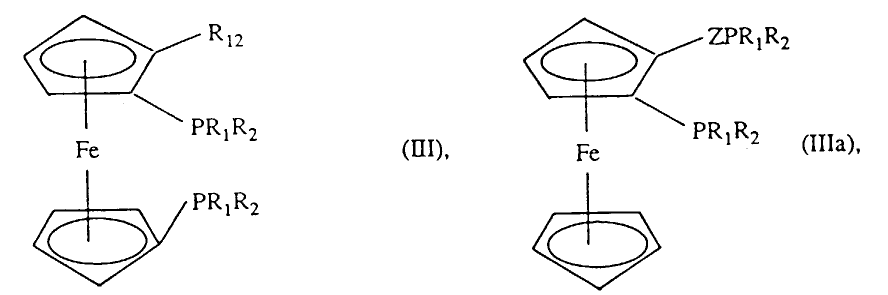 where R₁ and R₂ are each independently of the other C₁-C₁₂alkyl, C₅cycloalkyl or C₆cycloalkyl, phenyl or benzyl which are unsubstituted or substituted by C₁-C₄alkyl or C₁-C₄alkoxy, R₁₂ is H, C₁-C₆alkyl, C₁-C₆alkenyl, tri(C₁-C₆alkyl)silyl, phenyl, benzyl, α-hydroxy- or α-[di(C₁-C₆alkyl)amino]-C₁-C₆alkyl, and Z is methylene or C₁-C₈-alkylidene.

43. A process according to claim 36, wherein the bidentate ligands are α,α'-bipyridyl which is unsubstituted or substituted by C₁-C₄alkyl, C₁-C₄alkoxy, F or Cl.

44. A process according to claim 36, wherein the bidentate ligands are bisoxazolidines of formula IV, 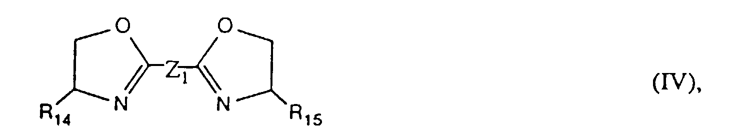 where R₁₄ and R₁₅ are identical or different and are H, linear or branched C₁-C₆alkyl, phenyl, (C₁-C₆alkyl)phenyl, benzyl or (C₁-C₆alkyl)benzyl, and Z₁ is a direct bond, methylene, C₂-C₈alkylidene, ethylene, 1,2-propylene or 1,2-phenylene.

45. A process according to claim 36, wherein the bidentate ligands are bisoxazolidines of formula V, 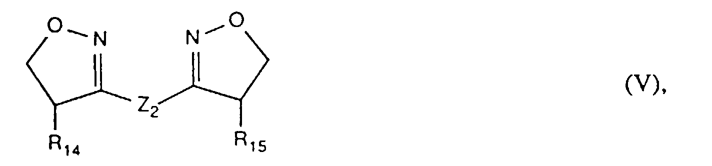 where R₁₄ and R₁₅ are identical or different and are H, linear or branched C₁-C₆alkyl, phenyl, (C₁-C₆alkyl)phenyl, benzyl or (C₁-C₆alkyl)benzyl, and Z₂ is a direct bond, methylene, 1,2- or 1,3-phenylene, pyridine-2,6-diyl, ethylene, 1,2- or 1,3-propylene or 1,2-, 1,3-, 1,4- or 2,3-butylene or C₂-C₈alkylidene.

46. A process according to claim 1, wherein the metal complex salts contain two different strongly co-ordinating bidentate ligands, one bidentate strongly co-ordinating ligand and one monodentate strongly co-ordinating ligand, one bidentate strongly co-ordinating ligand and two monodentate weakly or strongly co-ordinating ligands, two different tridentate strongly co-ordinating ligands or one tridentate strongly co-ordinating ligand and three monodentate weakly or strongly co-ordinating ligands.

47. A process according to claim 1, wherein the metal complex salts contain a halide as nucleophilic co-ordinating anions.

48. A process according to claim 1, wherein the metal complex salts contain sulfate, phosphate, perchlorate, perbromate, periodate, antimonate, arsenate, nitrate, carbonate, the anion of a C₁-C₈carboxylic acid, sulfonates and phosphonates as anions of oxyacids.

49. A process according to claim 1, wherein the metal complex salts contain tetrafluoroborate, hexafluorophosphate, perchlorate or triflate as anions.

50. A process according to claim 1, wherein the metal complex salts have the formula VI,
[(Q)ₘ(M⁺ⁿ)(S)ₒ(L⁻¹)_{q}]^{+(n-q)}(A^{-r})_{(n-q)/r} (VI),
where Q denotes identical or different strongly co-ordinating ligands, m is 0, or Q is a monodentate ligand and m is a number from 1 to 8, Q is a bidentate ligand and m is a number from 1 to 4, Q is a tridentate ligand and m is 1 or 2, or Q is a tetradentate ligand and m is 1 or 2, the bidentate, tridentate and tetradentate ligands containing one or more identical or different phosphino, phosphonite, arsino or stibino groups or primary, secondary and/or tertiary amino groups and/or imino groups as complex-forming groups, and these ligands forming with the metal cation M⁺ⁿ a 5- to 7-membered ring, preferably a 5- or 6-membered ring; M is a metal selected from the second to fifth main group, from the first to the eighth subgroup, or from the lanthanides, of the Periodic Table of the Elements, and n is a number from 1 to 4; S is a weakly co-ordinating ligand selected from the group consisting of nitriles, aliphatic or aromatic alcohols, ketones, aldehydes, carboxylates, ethers, tris(trifluoromethyl) phosphite, and water, and o is 0 or a number from 1 to 6; L is halide, pseudohalide, C₁-C₈alcoholate or unsubstituted or C₁-C₄alkylsubstituted phenolate, secondary amide of 2 to 12 carbon atoms, bis[(tri-C₁-C₆alkyl)silyl]amide or unsubstituted or C₁-C₄alkyl-substituted cyclopentadienyl, and q is 0 or a number from 1 to 4; A is an anion of an oxyacid, BF₄, PF₆, AsF₆ or SbF₆, and r is a number from 1 to 3, where the sum of (m x denticity of the ligand Q) + o + q is an integer from 2 to 8 and the sum of (m x the denticity of the ligand Q) + o is at least 2.

51. A process according to claim 1, wherein solvents used are halogenated aliphatic hydrocarbons, aromatic hydrocarbons, ethers and nitriles.

52. A process according to claim 1, wherein the protected sugar and the aglycon are used in equimolar amounts.

53. A process according to claim 1, wherein the orthoester is used in an amount of from 1.2 to 10 equivalents per equivalent of protected sugar.

54. A process according to claim 1, wherein the amount is from 1.2 to 5 equivalents.

55. A process according to claim 1, wherein the reaction temperature is from -20 to 250°C.

56. A process according to claim 1, wherein the reaction temperature is from 10 to 200°C.

57. A process according to claim 1, wherein an orthoester is used concurrently and the reaction is carried out at room temperature.

58. A process according to claim 1, wherein the water of reaction formed is bound chemically or is removed by distillation or by the use of water-absorbents.

59. A process according to claim 1, wherein the protected sugar and the catalyst are charged in a solvent at room temperature to the reactor and then the aglycon and an orthoester are added, and then the mixture is stirred at room temperature up to the reflux temperature of the solvents.

60. A process according to claim 59, wherein orthoesters of alcohols are used which are employed simultaneously as aglycon.

61. A process according to claim 1, wherein the protected sugar and the aglycon are charged in a solvent to the reactor and then the catalyst is added, the water-absorbent is charged in a Soxhlet apparatus in a jacket, and then the reaction mixture is heated under reflux.

62. A process according to claim 1, wherein the protected sugar and the aglycon are charged in a solvent to the reactor and then the catalyst is added, using an apparatus with water separator, then the reaction mixture is heated under reflux and the water of reaction is distilled off azeotropically until the reaction is at an end.

## Revendications

1. Procédé de préparation de glucosides par réaction de sucres ayant un groupe hydroxyle anomère et d'une aglucone, caractérisé en ce que l'on fait réagir, dans un solvant inerte, un sucre protégé qui contient un groupe hydroxyle anomère, avec une aglucone du groupe a) des alcools aliphatiques, alcools cycloaliphatiques, alcools aromatiques ou aromatico-aliphatiques, et b) des sucres protégés ayant un groupe hydroxyle non anomère soit chacun seul, soit chacun ensemble avec un orthoester, en présence de quantités catalytiques d'un sel métallique complexe constitué de (1) un cation métallique d'un métal des groupes principaux deux à cinq, des sous-groupes un à huit ou des lanthanides de la Classification Périodique des Eléments, (2) un ou plusieurs ligands mono- ou polydentés, identiques ou différents, correspondant à la coordinence du cation métallique, (3) éventuellement un anion nucléophile du groupe halogénure, pseuso-halogénure, alcoolate en C₁-C₈ ou phénolate non substitué ou substitué par alkyle en C₁-C₄, amido secondaire avec 2 à 12 atomes de C, bis-[(tri-alkyl en C₁-C₆)silyl] amido ou cyclopentadiényle non substitué ou substitué par alkyle en C₁-C₄, et (4) un anion non nucléophile du groupe constitué par un anion d'un acide oxygéné, BF₄, PF₆, AsF₆ ou SbF₆, selon la valence du cation métallique complexé.

2. Procédé selon la revendication 1, caractérisé en ce que les sucres protégés sont des mono- et oligosaccharides protégés.

3. Procédé selon la revendication 2, caractérisé en ce que les mono- et oligosaccharides protégés sont des aldoses ou des cétoses avec un groupe hydroxyle anomère.

4. Procédé selon la revendication 3, caractérisé en ce que les monosaccharides protégés sont des aldopyranoses, des aldofuranoses, des cétopyranoses ou des cétofuranoses avec un groupe hydroxyle anomère.

5. Procédé selon la revendication 1, caractérisé en ce que les dérivés de mono- et d'oligosaccharides protégés sont des désoxysucres, aminosucres, thiosucres, acides sacchariques ou esters d'acides sacchariques protégés qui contiennent un groupe hydroxyle anomère.

6. Procédé selon la revendication 1, caractérisé en ce que les orthoesters dérivent d'un acide carboxylique en C₁-C₄.

7. Procédé selon la revendication 6, caractérisé en ce que les orthoesters dérivent de l'acide formique, de l'acide acétique, de l'acide propionique ou de l'acide butyrique.

8. Procédé selon la revendication 7, caractérisé en ce que les orthoesters dérivent de l'acide formique.

9. Procédé selon la revendication 6, caractérisé en ce que les orthoesters dérivent d'un acide carboxylique en C₁-C₄ et d'un alcanol en C₁-C₂₀ linéaire ou ramifié, d'un alcénol en C₂-C₂₀ à groupe alcool non vinylique, d'un alcool en C₃-C₃₀ mono- ou polycyclo-aliphatique ou mono- ou polycyclohétéroaliphatique, avec les hétéroatomes O, S et N, d'un alcool aromatique en C₆-C₂₀, ou d'un alcool aromatico-aliphatique en C₇-C₂₀, qui sont non substitués ou substitués par alkyle en C₁-C₆, alcoxy ou alkylthio en C₁-C₆, phényloxy, (alkyl en C₁-C₄)Phényloxy ou (alcoxy en C₁-C₄)phényloxy, benzyle, benzyloxy ou (alkyl en C₁-C₄)benzyloxy ou (alcoxy en C₁-C₄)benzyloxy.

10. Procédé selon la revendication 9, caractérisé en ce que les esters de l'acide orthoformique sont des esters d'alcanols en C₁-C₄, d'alcool allylique, d'alcool benzylique et de phénol.

11. Procédé selon la revendication 1, caractérisé en ce que les aglucones b) sont des sucres ou des dérivé de sucres protégés dont le groupe hydroxyle anomère est protégé et qui contiennent un groupe hydroxyle libre.

12. Procédé selon la revendication 11, caractérisé en ce que les sucres ou les dérivés de sucres contiennent un groupe hydroxyméthyle libre.

13. Procédé selon la revendication 1, caractérisé en ce que les sels métalliques complexes sont utilisés en une quantité de 0,01 à 20 moles %, par rapport à la quantité du sucre ou du dérivé de sucre protégé.

14. Procédé selon la revendication 13, caractérisé en ce que les sels métalliques complexes sont utilisés en une quantité de 0,01 à 10 moles %, par rapport à la quantité du sucre ou du dérivé de sucre protégé.

15. Procédé selon la revendication 1, caractérisé en ce que les cations métalliques dérivent des métaux suivants des groupes cités de la Classification Périodique des Eléments : Mg, Ca, Sr, Ba ; B, Al, Ga, In ; Sn, Pb ; Sb, Bi ; Cu, Ag, Au ; Zn, Cd, Hg ; Sc, Y, La ; Ti, Zr, Hf ; V, Nb, Ta ; Cr, Mo, W ; Mn ; Fe, Co, Ni, Ru, Rh, Pd, Os, Ir, Pt ; et des métaux des lanthanides Ce, Pr, Nd, Pm, Sm, Eu, Gd, Tb, Dy, Ho, Er, Tm, Yb et Lu.

16. Procédé selon la revendication 15, caractérisé en ce que les cations métalliques dérivent des métaux suivants : Mg, Ca, B, In, Sn, Pb, Cu, Ag, Au, Zn, Ti, Zr, V, Cr, Mo, W, Mn, Fe, Co, Ni, Ru, Rh, Pd, Ir, Pt, Ce, Nd et Yb.

17. Procédé selon la revendication 1, caractérisé en ce que le ligand est achiral ou chiral.

18. Procédé selon la revendication 1, caractérisé en ce que le ligand est mono- à tridenté.

19. Procédé selon la revendication 1, caractérisé en ce que les ligands polydentés forment avec les groupes complexants et avec le cation métallique un cycle à 5 à 7 membres.

20. Procédé selon la revendication 1, caractérisé en ce que les groupes complexants des ligands polydentés sont liés en positions 1,2, 1,3 ou 1,4 d'une chaîne carbonée avec 2 à 4 atomes de C.

21. Procédé selon la revendication 1, caractérisé en ce que les ligands sont des ligands organiques faiblement coordonnants avec 1 à 20, de préférence 1 à 12 atomes de C qui contiennent des hétéroatomes ou des hétérogroupes aptes à la coordination, ou sont H₂O.

22. Procédé selon la revendication 21, caractérisé en ce que les hétéroatomes ou les hétérogroupes sont -OH, -CN, -CHO, -CO-, -O-, -C(O)OR₀ et P(O-)₃, où R₀ est le radical d'un alcool en C₁-C₁₂.

23. Procédé selon la revendication 21, caractérisé en ce que les ligands organiques faiblement coordonnants sont des alcools avec 1 à 12 atomes de C, des nitriles, des isonitriles, des aldéhydes, des cétones, des esters d'alkyle en C₁-C₄ et d'acides carboxyliques aliphatiques, des éthers ou des phosphites.

24. Procédé selon la revendication 23, caractérisé en ce que les ligands organiques faiblement coordonnants sont l'acétonitrile, le benzonitrile, le méthanol, l'éthanol, le phénol, l'acétone, l'aldéhyde pivalique, l'acétate d'éthyle, l'éther diéthylique, le tétrahydrofuranne et le dioxane, ainsi que l'eau.

25. Procédé selon la revendication 1, caractérisé en ce que les sels métalliques complexes ne contiennent que des ligands faiblement coordonnants, que des ligands fortement coordonnants ou contiennent les deux types de ligands.

26. Procédé selon la revendication 1, caractérisé en ce que les ligands fortement coordonnants sont des composés organiques qui contiennent 2 à 30 atomes de C, et en tant que groupes complexants contiennent un ou plusieurs groupes amino, phosphino, phosphonite, arsino ou stibino éventuellement substitués, identiques ou différents.

27. Procédé selon la revendication 26, caractérisé en ce que les groupes amino,phosphino, phosphonite, arsino ou stibino sont substitués par alkyle en C₁-C₁₂, cycloalkyle en C₅ ou C₆, phényle ou benzyle, qui sont eux-mêmes non substitués ou substitués par alkyle en C₁-C₄ ou alcoxy en C₁-C₄.

28. Procédé selon la revendication 21, caractérisé en ce que les ligands fortement coordonnants monodentés sont des amines tertiaires, des phosphines, des arsines ou des stibines.

29. Procédé selon la revendication 28, caractérisé en ce que les ligands fortement coordonnants monodentés sont des ligands de formule XR₁R₂R₃, dans laquelle X représente N, P, As ou Sb et R₁, R₂ et R₃, indépendamment l'un de l'autre, représentent alkyle en C₁-C₁₂, cycloalkyle en C₅ ou C₆, phényle ou benzyle, ou R₁ et R₂ représentent ensemble tétraméthylène, pentaméthylène ou 3-oxa-1,5-pentylène et R₃ a la signification indiquée précédemment, lesquels sont non substitués ou substitués par alkyle en C₁-C₄ ou alcoxy en C₁-C₄.

30. Procédé selon la revendication 28, caractérisé en ce que les ligands fortement coordonnants monodentés sont des phosphines tertiaires.

31. Procédé selon la revendication 1, caractérisé en ce que les groupes complexants dans les ligands polydentés fortement coordonnants correspondent aux formules -NR₁R₂, -PR₁R₂, -OPR₁R₂, -AsR₁R₂ ou -SbR₁R₂, où R₁ et R₂ sont, indépendamment l'un de l'autre, alkyle en C₁-C₁₂, cycloalkyle en C₅ ou C₆, phényle ou benzyle, qui sont non substitués ou substitués par alkyle en C₁-C₄ ou alcoxy en C₁-C₄.

32. Procédé selon la revendication 31, caractérisé en ce que le groupe complexant correspond à la formule -PR₁R₂.

33. Procédé selon la revendication 1, caractérisé en ce que les ligands fortement complexants tridentés sont des phosphines de formule R₄C(CH₂PR₁R₂)₃, dans laquelle R₁ et R₂ ont les significations données dans la revendication 31, et R₄ représente H, alkyle en C₁-C₄, phényle ou benzyle, ou sont des diéthylènetriamines N,N',N"'-pentaalkylées, les groupes alkyle contenant 1 à 4 atomes de C.

34. Procédé selon la revendication 33, caractérisé en ce que les ligands fortement complexants tridentés sont CH₃-C[CH₂P(C₆H₅)₂]₃, la bis-[(2-diphénylphosphino)-éthyl]phénylphosphine ou la N,N',N"-pentaméthyldiéthylènetriamine.

35. Procédé selon la revendication 1, caractérisé en ce que les ligands fortement complexants tétradentés sont C[CH₂P(C₆H₅)]₄, la tri[(2-diphénylphosphino)-éthyl]phosphine, la tri[(2-diméthylaminoéthyl]amine ou la N,N',N",N"'-hexaméthyl-triéthylènetétraamine.

36. Procédé selon la revendication 1, caractérisé en ce que les ligands fortement complexants bidentés sont des composés aliphatiques, cycloaliphatiques, cyclohétéroaliphatiques, aromatiques ou hétéroaromatiques avec 2 à 30 (atomes de C) (sans les atomes de C dans les groupes complexants), qui contiennent des groupes complexants identiques ou différents, les groupes complexants dans les composés aliphatiques étant liés en position 1,2, 1,3 ou 1,4 et dans les composés cycliques en position 1,2 ou 1,3, et les hétéroatomes étant choisis dans le groupe constitué par O, S et N et un ou deux hétéroatomes étant présents, et les groupes complexants étant liés à la charpente de base directement ou par l'intermédiaire d'un groupe -CR₅R₆, et R₅ et R₆, indépendamment l'un de l'autre, représentent H, alkyle en C₁-C₄, phényle ou benzyle.

37. Procédé selon la revendication 36, caractérisé en ce que les groupes complexants sont des groupes de formules -NR₁R₂, -PR₁R₂, -OPR₁R₂, -AsR₁R₂ ou -SbR₁R₂, dans lesquelles R₁ et R₂ sont, indépendamment l'un de l'autre, alkyle en C₁-C₁₂, cycloalkyle en C₅ ou C₆, phényle ou benzyle, qui sont non substitués ou substitués par alkyle en C₁-C₄ ou alcoxy en C₁-C₄.

38. Procédé selon la revendication 36, caractérisé en ce que les ligands bidentés correspondent à la formule I,
R₁R₂X-Y-X'R₁R₂ (I)
dans laquelle X et X', indépendamment l'un de l'autre, représentent N, P, As, Sb ou -OP, R₁ et R₂ représentent, indépendamment l'un de l'autre, alkyle en C₁-C₁₂, cycloalkyle en C₅ ou C₆, phényle ou benzyle, qui sont non substitués ou substitués par alkyle en C₁-C₄ ou alcoxy en C₁-C₄, Y représente un radical alkylène ou alcénylène avec 2 à 20 atomes de C, linéaire ou ramifié, qui est non substitué ou substitué par alcoxy en C₁-C₆, cycloalkyle en C₅ ou C₆, cycloalcoxy en C₅ ou C₆, phényle, phényloxy, benzyle ou benzyloxy, et les substituants cycliques sont eux-mêmes non substitués ou substitués par alkyle en C₁-C₆ ou alcoxy en C₁-C₆, et les groupes R₁R₂X- ou respectivement R₁R₂X'- dans le radical alkylène ou dans le radical alcénylène sont liés en position α,β, α,γ ou α,δ.

39. Procédé selon la revendication 36, caractérisé en ce que les ligands bidentés correspondent à la formule II, 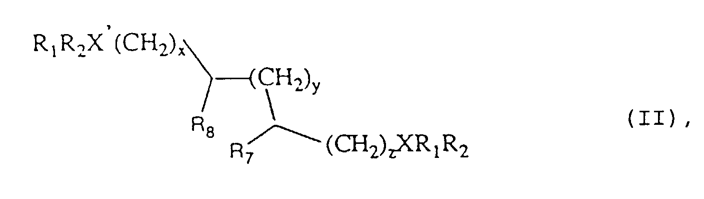 dans laquelle X et X' représentent, indépendamment l'un de l'autre, N, P, As, Sb ou -OP, R₁ et R₂ représentent, indépendamment l'un de l'autre, alkyle en C₁-C₁₂, cycloalkyle en C₅ ou C₆, phényle ou benzyle, qui sont non substitués ou substitués par alkyle en C₁-C₄ ou alcoxy en C₁-C₄, x, y et z représentent, indépendamment l'un de l'autre, 0 ou 1, la somme de x+y+z étant de 0, 1 ou 2, R₇ et R₈ forment, ensemble avec le radical auquel ils sont liés, un radical cycloaliphatique à 5 ou 6 membres ou un radical cyclohétéroaliphatique à 5 ou 6 membres avec 1 ou 2 hétéroatomes identiques ou différents choisis dans le groupe constitué par O, S et NR₉, qui sont non substitués ou substitués par alkyle en C₁-C₆ ou alcoxy en C₁-C₆ ou halogène, R₉ représente H, alkyle en C₁-C₆, acyle en C₁-C₈, ou phényle ou benzyle non substitué ou substitué par alkyle en C₁-C₆ ou alcoxy en C₁-C₆, et si y représente 0, R₇ et R₈ forment, ensemble avec le radical auquel ils sont liés, un radical aromatique ou hétéroaromatique à 6 membres ou un radical hétéroaromatique à 5 membres qui sont non substitués ou substitués par alkyle en C₁-C₆ ou alcoxy en C₁-C₆.

40. Procédé selon la revendication 1, caractérisé en ce que les ligands fortement complexants bidentés sont le groupe biphénylène ou naphtylène substitué en positions o,o' par des groupes XR₁R₂ identiques ou différents, dans lesquels les X représentent, indépendamment les uns des autres, N, P, As, Sb ou -OP, R1 et R2 sont, indépendamment l'un de l'autre, alkyle en C₁-(C₁₂)-C₆, cycloalkyle en C₅ ou C₆, phényle ou benzyle, qui sont non substitués ou substitués par alkyle en C₁-C₄ ou alcoxy en C₁-C₄.

41. Procédé selon la revendication 40, caractérisé en ce qu'il s'agit du 2,2'-diphénylphosphino- ou -dicyclohexylphosphinobiphényle ou -binaphtyle.

42. Procédé selon la revendication 36, caractérisé en ce que les ligands bidentés correspondent à la formule II, 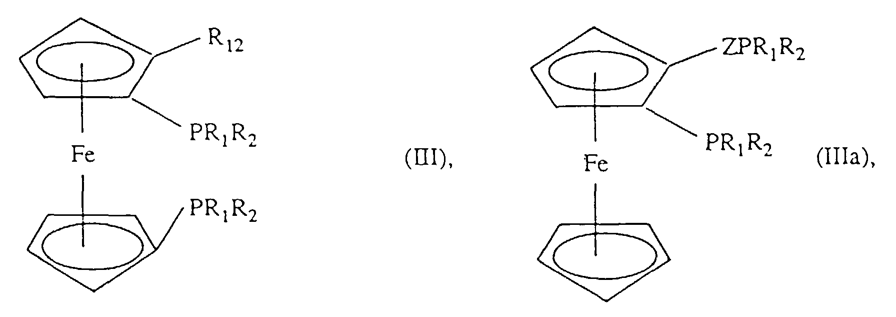 où R₁ et R₂, indépendamment l'un de l'autre, représentent alkyle en C₁-C₁₂, cycloalkyle en C₅ ou C₆, phényle ou benzyle, qui sont non substitués ou substitués par alkyle en C₁-C₄ ou alcoxy en C₁-C₄, R₁₂ représente H, alkyle en C₁-C₆, alcényle en C₁-C₆, tri(alkyl en C₁-C₆)silyle, phényle, benzyle, α-hydroxy- ou α-[di(alkyl en C₁-C₆)amino]-alkyle en C₁-C₆, et Z représente méthylène, ou alkylidène en C₁-C₈.

43. Procédé selon la revendication 36, caractérisé en ce que les ligands bidentés sont l'α,α'-bipyridyle non substitué ou substitué par alkyle en C₁-C₄, alcoxy en C₁-C₄, F ou Cl.

44. Procédé selon la revendication 36, caractérisé en ce que les ligands bidentés sont des bisoxazolidines de formule IV, 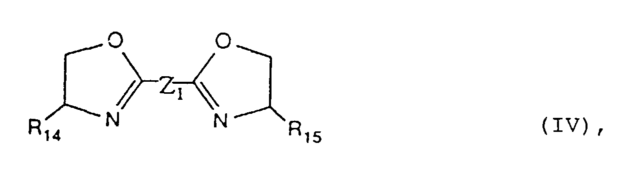 dans laquelle R₁₄ et R₁₅ sont identiques ou différents et représentent H, alkyle en C₁-C₆ linéaire ou ramifié, phényle, (alkyl en C₁-C₆)phényle, benzyle ou (alkyl en C₁-C₆)benzyle, et Z₁ représente une liaison directe, méthylène, alkylidène en C₂-C₈, éthylène, 1,2-propylène ou 1,2-phénylène.

45. Procédé selon la revendication 36, caractérisé en ce que les ligands bidentés sont des bisoxazolidines de formule V, 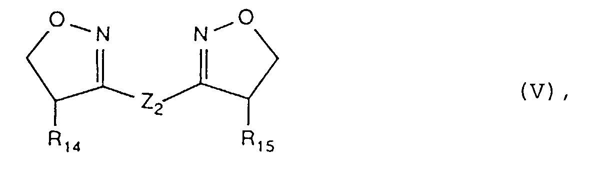 dans laquelle R₁₄ et R₁₅ sont identiques ou différents et représentent H, alkyle en C₁-C₆ linéaire ou ramifié, phényle, (alkyl en C₁-C₆)phényle, benzyle ou (alkyl en C₁-C₆)benzyle, et Z₂ représente une liaison directe, méthylène, 1,2- ou 1,3-phénylène, pyridine-2,6-diyle, éthylène, 1,2- ou 1,3-propylène, ou 1,2-, 1,3-, 1,4 ou 2,3-butylène, ou alkylidène en C₂-C₈.

46. Procédé selon la revendication 1, caractérisé en ce que les sels métalliques complexes contiennent deux ligands bidentés fortement coordonnants différents, un ligand bidenté fortement coordonnant et un ligand monodenté fortement coordonnant, un ligand bidenté fortement coordonnant et deux ligands monodentés faiblement ou fortement coordonnants, deux ligands tridentés fortement coordonnants différents ou un ligand tridenté fortement coordonnant et trois ligands monodenté faiblement ou fortement coordonnants.

47. Procédé selon la revendication 1, caractérisé en ce que les sels métalliques complexes contiennent, en tant qu'anions coordonnants nucléophiles, un halogénure.

48. Procédé selon la revendication 1, caractérisé en ce que les sels métalliques complexes contiennent, en tant qu'anions d'acides oxygénés, du sulfate, du phosphate, du perchlorate, du perbromate, du periodate, de l'antimonate, de l'arsénate, du nitrate, du carbonate, l'anion d'un acide carboxylique en C₁-C₈, des sulfonates et des phosphonates.

49. Procédé selon la revendication 1, caractérisé en ce que les sels métalliques complexes contiennent, en tant qu'anions, du tétrafluoroborate, de l'hexafluorophosphate, du perchlorate ou du triflate.

50. Procédé selon la revendication 1, caractérisé en ce que les sels métalliques complexes correspondent à la formule VI,
[(Q)ₘ(M⁺ⁿ)(S)ₒ(L⁻¹)_{q}]^{+(n-q)}(A^{-r})_{(n-q)/r} (VI),
dans laquelle Q représente des ligands fortement coordonnants, identiques ou différents, m représente 0 ou Q est un ligand monodenté et m un nombre de 1 à 8, Q est un ligand bidenté et m un nombre de 1 à 4, Q est un ligand tridenté et m les nombres 1 ou 2 ou Q est un ligand tétradenté et m les nombres 1 ou 2, les ligands bi-, tri- et tétradentés contenant un ou plusieurs groupes phosphine, phosphonite, arsine ou stibine, identiques ou différents ou des groupes amine et/ou imine primaires, secondaires et/ou tertiaires en tant que groupes complexants, et ces ligands formant, avec le cation métallique M⁺ⁿ un cycle à 5 à 7 membres, de préférence à 5 ou 6 membres ; M représente un métal des groupes principaux 2 à 5, des sous-groupes 1 à 8 ou des lanthanides dans la Classification Périodique des Eléments et n est un nombre de 1 à 4 ; S représente un ligand faiblement coordonnant du groupe constitué par les nitriles, les alcools aliphatiques ou aromatiques, les cétones, les aldéhydes, les esters d'acides carboxyliques, les éthers, le phosphite de tris(trifluorométhyle) et l'eau, et o représente 0 ou un nombre de 1 à 6 ; L représente halogénure, pseudohalogénure, alcoolate en C₁-C₈ ou phénolate non substitué ou substitué par alkyle en C₁-C₄, amide secondaire avec 2 à 12 atomes de C, bis[(tri-alkyl en C₁-C₆)silyl]amide ou cyclopentadiényle non substitué ou substitué par alkyle en C₁-C₄ et q représente 0 ou un nombre de 1 à 4 ; A représente un anion d'un acide oxygéné, BF₄, PF₆, AsF₆ ou SbF₆ et r représente un nombre de 1 à 3, la somme de (m × denture du ligand Q) + o + q étant un nombre entier de 2 à 8 et la somme de (m × denture du ligand Q) + o étant au moins 2.

51. Procédé selon la revendication 1, caractérisé en ce que l'on utilise comme solvants des hydrocarbures aliphatiques halogénés, des hydrocarbures aromatiques, des éthers et des nitriles.

52. Procédé selon la revendication 1, caractérisé en ce que le sucre protégé et l'aglucone sont utilisés en quantités équimolaires.

53. Procédé selon la revendication 1, caractérisé en ce que l'orthoester est mis en oeuvre en une quantité de 1,2 à 10 équivalents par équivalent de sucre protégé.

54. Procédé selon la revendication 1, caractérisé en ce que la quantité est de 1,2 à 5 équivalents.

55. Procédé selon la revendication 1, caractérisé en ce que la température de réaction est de -20 à 250 °C.

56. Procédé selon la revendication 1, caractérisé en ce que la température de réaction est de 10 à 200 °C.

57. Procédé selon la revendication 1, caractérisé en ce que l'on utilise simultanément un orthoester et on effectue la réaction à la température ambiante.

58. Procédé selon la revendication 1, caractérisé en ce que l'on fixe chimiquement l'eau de réaction formée ou en ce qu'on l'élimine par distillation ou en utilisant des agents absorbant l'eau.

59. Procédé selon la revendication 1, caractérisé en ce que l'on introduit préalablement le sucre protégé et le catalyseur dans un solvant à la température ambiante, puis on ajoute l'aglucone et un orthoester, puis on agite le mélange à la température ambiante jusqu'à la température de reflux du solvant.

60. Procédé selon la revendication 59, caractérisé en ce que l'on utilise des orthoesters d'alcools qui sont utilisés en même temps comme aglucone.

61. Procédé selon la revendication 1, caractérisé en ce que l'on introduit au préalable le sucre protégé et l'aglucone dans un solvant, puis on ajoute le catalyseur, on dispose l'agent absorbant l'eau dans un appareil de Soxhlet dans un manchon, puis on chauffe le mélange réactionnel au reflux.

62. Procédé selon la revendication 1, caractérisé en ce que l'on introduit au préalable le sucre protégé et l'aglucone dans un solvant, puis on ajoute le catalyseur, en utilisant un appareillage avec séparateur d'eau, puis on chauffe le mélange réactionnel au reflux et on chasse l'eau de réaction par distillation azéotrope jusqu'à la fin de la réaction.
